(19) Europäisches Patentamt
European Patent Office
Office européen des brevets

(11) Numéro de publication: **0 484 223 B1**

(12) # FASCICULE DE BREVET EUROPEEN

(45) Date de publication de fascicule du brevet: **08.03.95**

(51) Int. Cl.[6]: **C07C 255/31**, A61K 31/275, C07C 255/19

(21) Numéro de dépôt: **91402890.7**

(22) Date de dépôt: **29.10.91**

(54) **Nouveaux 3-cycloalkyl-propanamides, leurs formes tautomères et leurs sels, procédé de préparation, application à titre de médicaments et compositions les renfermant.**

(30) Priorité: **30.10.90 GB 9023535**
**15.03.91 GB 9105516**

(43) Date de publication de la demande:
**06.05.92 Bulletin 92/19**

(45) Mention de la délivrance du brevet:
**08.03.95 Bulletin 95/10**

(84) Etats contractants désignés:
**AT BE CH DE DK ES FR GB GR IT LI LU NL SE**

(56) Documents cités:
**EP-A- 0 326 107**
**DE-A- 2 555 789**

**Chemical Abstracts, 9th Collective Index, Volumes 76-85, 1972-1976, Chemical Substances, 1978, Columbus, Ohio, US**

**Chemical Abstracts, 10th Collective Index, Volumes 86-95, 1977-1981, Chemical Substances, 1982, Columbus, Ohio, US**

(73) Titulaire: **ROUSSEL-UCLAF**
**35, Boulevard des Invalides**
**F-75007 Paris (FR)**

(72) Inventeur: **Hambleton, Philip Thomas**
**24 Bullfinch Close**
**Covingham,**
**Swindon,**
**Wiltshire SN3 5HP (GB)**
Inventeur: **Hedgecock, Charles John Robert**
**19 Longleaze,**
**Wootton Basset**
**Swindon,**
**Wiltshire SN4 8AX (GB)**
Inventeur: **Kay, David Paul**
**4 Church Path**
**Purton,**
**Swindon,**
**Wiltshire SN5 9DR (GB)**
Inventeur: **Kuo, Elizabeth Anne**
**8 Bullfinch Close**
**Covingham,**
**Swindon,**
**Wiltshire SN3 5PH (GB)**

**EP 0 484 223 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**Chemical Abstracts, 11th Collective Index, Volumes 96-105, 1982-1986, Chemical Substances, 1987, Columbus, Ohio, US**

**Chemical Abstracts, 12th Collective Index, Volumes 106-115, 1987-1991, Chemical Substances, 1992, Columbus, Ohio, US**

Inventeur: **Tully, Wilfred Roger**
**Abbey Cottage,**
**1, Grove Court,**
**The Waterloo**
**Cirencester,**
**Glos GL7 2PZ (GB)**

(74) Mandataire: **Jacobi, Markus Alexander et al**
**Roussel Uclaf**
**111, Route de Noisy**
**B.P. 9**
**F-93230 Romainville (FR)**

## Description

La présente invention concerne de nouveaux 3-Cycloalkyl-propanamides, leurs formes tautomères et leurs sels, ainsi que le procédé de préparation, l'application à titre de médicaments de ces nouveaux produits et les compositions les renfermant. Les documents DE-A 2555789 et EP-A 326107 décrivent des amides et leurs activités pharmacologiques.

L'invention a pour objet de nouveaux 3-Cycloalkyl-propanamides répondant à la formule générale (I) :

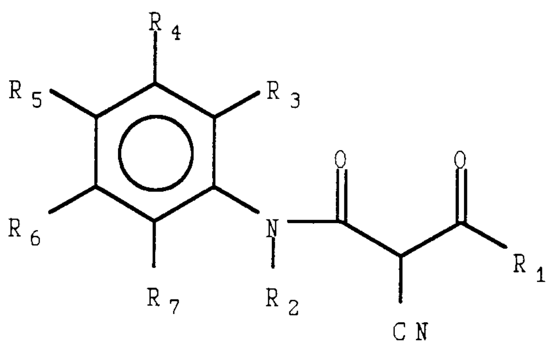

(I)

dans laquelle :

- $R_1$ représente un groupement cycloalkyle renfermant de 3 à 6 atomes de carbone,
- $R_2$ représente un atome d'hydrogène, un radical alkyle renfermant de 1 à 3 atomes de carbone,
- $R_3$, $R_4$, $R_5$, $R_6$ et $R_7$, identiques ou différents, représentent un atome d'hydrogène, un atome d'halogène, un radical alkyle linéaire ou ramifié renfermant de 1 à 6 atomes de carbone, un radical alcoxy linéaire ou ramifié renfermant de 1 à 6 atomes de carbone, un radical alkylthio renfermant de 1 à 6 atomes de carbone, un radical $-(CH_2)_m-CF_3$, $-O-(CH_2)_m-CF_3$, $-S-(CH_2)_m-CF_3$ dans lesquels m représente un nombre entier compris entre 0 et 3, un radical $-CF_2$-Hal, $-OCF_2$-Hal,

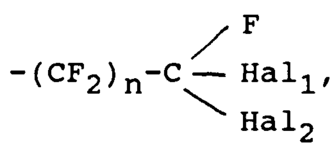

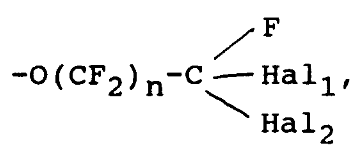

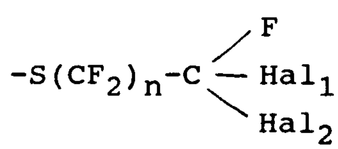

ou $O(CF_2)_n$-CF-$Hal_3$-$CF_3$, dans lequel n représente un nombre entier compris entre 1 et 3, $Hal_1$ et $Hal_2$ identiques ou différents, Hal et $Hal_3$ représentent un atome d'halogène,

ou $R_3$, $R_4$, $R_5$, $R_6$ et $R_7$ identiques ou différents représentent un groupement nitro, un groupement azido, un groupement nitrile, un groupement -CO-R′ dans lequel R′ représente un radical hydroxy, alkyle ou alcoxy renfermant de 1 à 3 atomes de carbone ou, $R_4$ et $R_5$ forment ensemble un groupement $-O-CH_2-O-$, leurs formes tautomères, ainsi que leurs sels d'addition avec les bases minérales ou organiques.

Dans la formule générale (I) et dans ce qui suit :

- par groupement cycloalkyle renfermant de 3 à 6 atomes de carbone, on entend un radical cyclopropyle, cyclobutyle, cyclopentyle ou cyclohexyle;
- par radical alkyle renfermant de 1 à 3 atomes de carbone, on entend un radical méthyle, éthyle, propyle, isopropyle;
- par radical alkyle renfermant de 1 à 6 atomes de carbone, on entend de préférence un radical méthyle, éthyle, propyle, isopropyle, butyle linéaire ou ramifié, pentyle linéaire ou ramifié, hexyle

linéaire ou ramifié,

- par radical alcoxy comportant de 1 à 6 atomes de carbone, on entend par exemple, un radical méthoxy, éthoxy, propoxy, isopropoxy, butoxy linéaire ou ramifié, pentyloxy linéaire ou ramifié, hexyloxy linéaire ou ramifié,
- par radical alkylthio comportant de 1 à 6 atomes de carbone, on entend par exemple, un radical méthylthio, éthylthio, propylthio, isopropylthio, butylthio linéaire ou ramifié, pentylthio linéaire ou ramifié, hexylthio linéaire ou ramifié,
- par atome d'halogène, on entend de préférence un atome de fluor, de chlore, de brome ou d'iode.

Les sels d'addition avec les bases minérales ou organiques peuvent être, par exemple, les sels formés avec les bases minérales tels que les sels de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris(hydroxyméthyl) aminométhane, l'éthanolamine, la pyridine, la picoline, la dicyclohexylamine, la morpholine, la benzylamine, la procaïne, la lysine, l'arginine, l'histidine, la N-méthylglucamine.

Parmi les produits, objet de l'invention, on peut citer notamment les dérivés répondant à la formule (I) ci-dessus, ainsi que leurs sels, caractérisés en ce que dans ladite formule (I), $R_3$, $R_4$, $R_5$, $R_6$ et $R_7$ identiques ou différents, représentent un atome d'hydrogène, un atome de fluor ,de chlore, de brome ou d'iode, un radical méthyle, éthyle, terbutyle, méthoxy, méthylthio, trifluorométhyle, trifluorométhoxy, trifluorométhylthio, pentafluoroéthyle, bromodifluorométhoxy, acétyle, hydroxycarbonyle, méthoxycarbonyle, nitro, azido, nitrile ou $R_4$ ou $R_5$ forment ensemble un groupement $O-CH_2-O-$, $R_2$ représente un atome d'hydrogène, ou un radical méthyle, $R_1$ a la signification déjà indiquée, ainsi que leurs sels d'addition avec les bases minérales ou organiques.

Parmi ces derniers, on peut citer plus particulièrement les dérivés répondant à la formule (I) ci-dessus, caractérisés en ce que dans ladite formule (I), $R_1$ représente un groupement cyclopropyle,
$R_2$ représente un atome d'hydrogène ou un radical méthyle, $R_3$, $R_4$, $R_5$, $R_6$ et $R_7$ identiques ou différents, représentent un atome d'hydrogène, un atome de fluor, de chlore ou d'iode, un radical méthyle, trifluorométhyle ou nitro, ainsi que leurs sels d'addition avec les bases minérales ou organiques.

Parmi ces derniers on retient tout particulièrement les dérivés de formule (I) dont les noms suivent :

- la 1-(4-nitrophénylcarbamoyl)-2-cyclopropyl-2-oxopropionitrile,
- la 1-(4-cyanophénylcarbamoyl)-2-cyclopropyl-2-oxopropionitrile,
- la 1-(4-chloro 3-méthylphénylcarbamoyl)-2-cyclopropyl-2-oxopropionitrile,
- la 1-(3-méthyl 4-trifluorométhylphenylcarbamoyl)-2-cyclobutyl-2-oxopropionitrile,
- le 1-(4-cyano 3-méthylphénylcarbamoyl) 2-cyclopropyl 2-oxopropionitrile,

ainsi que leurs sels d'addition avec les bases minérales ou organiques.

L'invention a également pour objet un procédé de préparation des nouveaux 3-cycloalkyl-propanamides tels que définis par la formule (I) ci-dessus, ainsi que de leurs sels, caractérisé en ce que l'on fait réagir un produit de formule (II) :

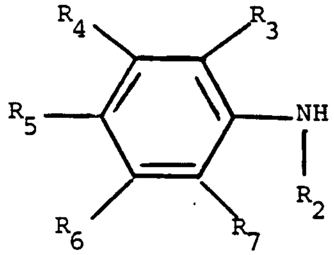

(II)

dans laquelle $R_2$, $R_3$, $R_4$, $R_5$, $R_6$ et $R_7$ ont la signification déja indiquée avec un acide de formule (III) ou un dérivé fonctionnel de cet acide :

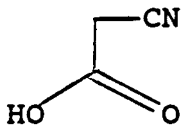

(III)

pour obtenir un produit de formule (IV) :

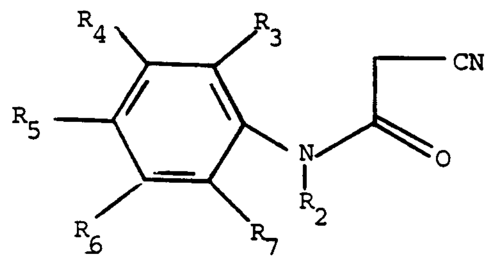

(IV)

dans laquelle $R_2$, $R_3$, $R_4$, $R_5$, $R_6$ et $R_7$ ont la signification déja indiquée, puis fait réagir ce dernier, successivement avec de l'hydrure de sodium, si nécessaire en présence d'un catalyseur tel que l'imidazole puis avec un produit de formule (V):

Hal-CO-$R_1$ (V)

dans laquelle Hal représente un atome d'halogène et $R_1$ a la signification déja indiquée, pour obtenir un produit de formule (I) correspondant que l'on isole et si désiré salifie.

Dans des conditions préférentielles de mise en oeuvre de l'invention, le procédé de préparation ci-dessus décrit est caractérisé en ce que :

- la réaction du produit de formule (II) avec l'acide de formule (III) ou un dérivé fonctionnel de cet acide est effectuée en présence de diisopropylcarbodiimide ou de dicyclohexylcarbodiimide au sein d'un solvant organique anhydre tel que le tétrahydrofuranne ou le dichlorométhane,
- le dérivé fonctionnel de l'acide de formule (III) peut être par exemple le chlorure de cyanoacétyle préparé in situ par action de l'acide cyanoacétique sur le pentachlorure de phosphore,
- la réaction du produit de formule (IV) avec l'hydrure de sodium est éffectuée au sein d'un solvant organique anhydre tel que le tétrahydrofuranne.

Les produits de formule (I) présentent un caractère acide. On peut avantageusement préparer les sels d'addition des produits de formule (I) en faisant réagir, en proportions sensiblement stoéchiométriques, une base minérale ou organique avec lesdits produits de formule (I). Les sels peuvent être préparés sans isoler les acides correspondants.

Les produits, objet de la présente invention, possèdent de très intéressantes propriétés pharmacologiques. On note en particulier une remarquable activité anti-inflammatoire. Ils inhibent d'une part les phénomènes inflammatoires provoqués par des agents irritants, et d'autre part les réactions d'hypersensibilité retardée, en empéchant l'activation des cellules immunitaires par un antigène spécifique.

Ces propriétés sont illustrées plus loin dans la partie expérimentale.

Ces propriétés justifient l'utilisation des nouveaux 3-Cycloalkyl-propanamides répondant à la formule (I), ainsi que de leurs sels d'addition avec les bases pharmaceutiquement acceptables, à titre de médicaments.

La présente invention a aussi également pour objet l'application à titre de médicaments des nouveaux 3-cycloalkyl-propanamides tels que définis par la formule générale (I), ainsi que de leurs sels d'addition avec les bases minérales ou organiques pharmaceutiquement acceptables.

Parmi les médicaments, objet de l'invention, on retient notamment les médicaments, caractérisés en ce qu'ils sont constitués par les nouveaux 3-cycloalkyl-propanamides répondant à la formule (I) dans laquelle $R_3$, $R_4$, $R_5$, $R_6$ et $R_7$ identiques ou différents, représentent un atome d'hydrogène, un atome de fluor ,de chlore, de brome ou d'iode, un radical méthyle, éthyle, terbutyle, méthoxy, méthylthio, trifluorométhyle, trifluorométhoxy, trifluorométhylthio, pentafluoroéthyle, bromodifluorométhoxy, acétyle, hydroxycarbonyle, méthoxycarbonyle, nitro, azido, nitrile ou, $R_4$ et $R_5$ forment ensemble un groupement $-O-CH_2-O-$, $R_2$ représente un atome d'hydrogène, ou un radical méthyle, $R_1$ a la signification déjà indiquée, ainsi que leurs sels d'addition avec les bases minérales ou organiques pharmaceutiquement acceptables.

Parmi les médicaments, objet de l'invention, on retient plus particulièrement ceux répondant à la formule (I) ci-dessus dans laquelle $R_1$ représente un groupement cyclopropyle, $R_2$ représente un atome d'hydrogène ou un radical méthyle, $R_3$, $R_4$, $R_5$, $R_6$ et $R_7$ identiques ou différents, représentent un atome d'hydrogène, un atome de fluor, de chlore ou d'iode, un radical méthyle, trifluorométhyle ou nitro, ainsi que leurs sels d'addition avec les bases minérales ou organiques pharmaceutiquement acceptables.

Parmi les médicaments préférés de l'invention, on retient tout particulièrement :

- la 1-(4-nitrophénylcarbamoyl)-2-cyclopropyl-2-oxopropionitrile,

- la 1-(4-cyanophénylcarbamoyl)-2-cyclopropyl-2-oxopropionitrile,
- la 1-(4-chloro 3-méthylphénylcarbamoyl)-2-cyclopropyl-2-oxopropionitrile,
- la 1-(3-méthyl 4-trifluorométhylphenylcarbamoyl)-2-cyclobutyl-2-oxopropionitrile,
- le 1-(4-cyano 3-méthylphénylcarbamoyl) 2-cyclopropyl 2-oxopropionitrile,

ainsi que leurs sels d'addition avec les bases minérales ou organiques pharmaceutiquement acceptables.

Ces médicaments trouvent, par exemple, leur emploi dans le traitement de l'arthrite rhumatoïde et des maladies inflammatoires chroniques d'origine immune ou non (greffes, transplantations d'organes, etc...).

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les bases pharmaceutiquement acceptables à titre de principe actif.

A titre de médicaments, les dérivés répondant à la formule (I) et leurs sels d'addition avec les bases pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet, à titre de produits industriels nouveaux et notamment à titre de produits industriels nécessaires comme intermédiaires pour la préparation des produits de formule (I) :

- les produits de formule (IV) :

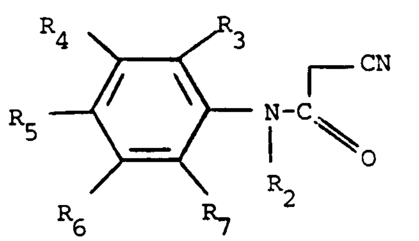

(IV)

dans laquelle $R_2$, $R_3$, $R_6$ et $R_7$ représentent un atome d'hydrogène, $R_4$ représente un radical méthyle et $R_5$ représente un groupe trifluorométhyle.

Ils peuvent être préparés selon un mode opératoire analogue à celui décrit par A. Nohara, T. Ishiguro et al dans J. Med. Chem. (1985) 28 (5), 559-566 selon le schéma suivant :

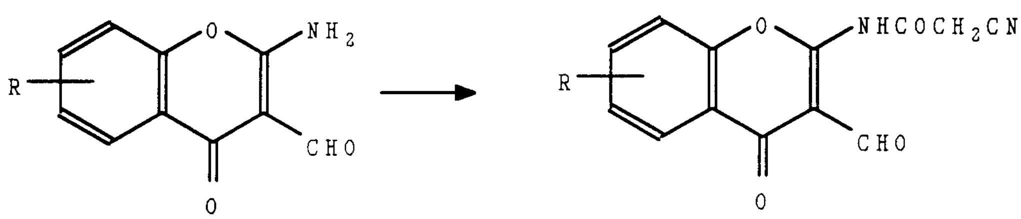

Les produits de formule (II) utilisés au départ du procédé sont en général des produits connus ou peuvent être préparés par diazotation puis réduction des nitroanilines correspondantes selon un procédé connu de l'homme de métier.

Les nitroanilines utilisées peuvent être préparées comme indiqué par exemple dans TP. Sura et al. Synthetic communications (1988) 18 (16-17) 2161-5.

Certaines anilines de formle (II) peuvent être préparées selon le brevet européen EP. 206951 ou par réducion de nitrobenzènes correspondants qui sont connus en général.

Certains nitrobenzènes sont nouveaux et peuvent être préparés comme indiqué ci-après dans les exemples.

Il va être donné maintenant à titre non limitatif, des exemples de mise en oeuvre de l'invention.

**EXEMPLE 1 : 1-(4-trifluorométhylphénylcarbamoyl)-2-cyclopropyl-2-oxopropionitrile.**

STADE A : 4-trifluorométhyl cyanoacétanilide.

On dissout 8,6 g d'acide cyanoacétique et 13,5 $cm^3$ de 4-(trifluorométhyl) aniline dans 100 $cm^3$ de tétrahydrofuranne, ajoute en 10 minutes, sous agitation sans refroidir 16,4 $cm^3$ de diisopropylcarbodiimide. La température varie de 20° à 60°C pendant l'addition ; on agite le mélange 16 heures à température ambiante, filtre, évapore le solvant, reprend le résidu ambiante ; on filtre, lave avec de l'éthanol, du chlorure de méthylène et de l'hexane. On sèche sous pression réduite à 60°C pendant 3 heures et obtient 18,85 g de produit attendu. F = 195-196°C.

STADE B : 1-(4-trifluorométhylphénylcarbamoyl)-2-cyclopropyl-2-oxopropionitrile.

A 3 g de produit obtenu au stade A en suspension dans 100 $cm^3$ de tétrahydrofuranne, on ajoute 0,88 g d'hydrure de sodium et agite 30 minutes à température ambiante. On ajoute en 10 minutes 1,30 $cm^3$ de chlorure de cyclopropanecarbonyle et agite le mélange 16 heures à température ambiante. On ajoute 1 $cm^3$ d'eau, agite 10 minutes, acidifie avec de l'acide chlorhydrique 2N et extrait à l'acétate d'éthyle. On sèche la phase organique, évapore les solvants. On chauffe le résidu dans 15 $cm^3$ de chlorure de méthylène, dilue à l'éther et obtient 2,72 g de produit attendu. F = 212-213°C.

En opérant selon le mode opératoire indiqué ci-dessus à partir des composés appropriés, on a préparé les produits des exemples suivants :

**EXEMPLE 2 : 1-(3-chlorophénylcarbamoyl) 2-cyclopropyl 2-oxopropionitrile.**

**EXEMPLE 3 : 1-(4-trifluorométhylphénylcarbamoyl) 2-cyclobutyl 2-oxopropionitrile.**

**EXEMPLE 4 : 1-(4-trifluorométhylphénylcarbamoyl) 2-cyclopentyl 2-oxopropionitrile.**

**EXEMPLE 5 : 1-(4-fluorophénylcarbamoyl) 2-cyclopropyl 2-oxopropionitrile.**

**EXEMPLE 6 : 1-(4-chlorophénylcarbamoyl) 2-cyclopropyl 2-oxopropionitrile.**

**EXEMPLE 7 : 1-(4-bromophénylcarbamoyl) 2-cyclopropyl 2-oxopropionitrile.**

**EXEMPLE 9 : 1-(4-trifluorométhoxyphénylcarbamoyl) 2-cyclopropyl 2-oxopropionitrile.**

**EXEMPLE 10 : 1-(4-nitrophénylcarbamoyl) 2-cyclopropyl 2-oxopropionitrile.**

**EXEMPLE 11 : 1-(3,4-dichlorophénylcarbamoyl) 2-cyclopropyl 2-oxopropionitrile.**

**EXEMPLE 8 : 1-(4-iodophénylcarbamoyl) 2-cyclopropyl 2-oxopropionitrile.**

STADE : 4′-iodocyanoacétanilide.

On ajoute en 2 minutes 14,41 g d'acide cyanoacétique à une suspension comprenant 35,25 g de pentachlorure de phosphore dans 250 $cm^3$ de chlorure de méthylène sous agitation en maintenant à température ambiante. On chauffe au reflux 30 minutes, agite sous courant d'azote pendant 2 minutes, ajoute 24,75 g de 4-iodoaniline et chauffe 2 heures au reflux, refroidit, verse sur 300 $cm^3$ d'eau. On agite 1 heure, filtre, reprend le résidu dans une solution aqueuse de bicarbonate de sodium, filtre, lave le résidu solide à l'eau, à l'éthanol puis le sèche à 60°C sous pression réduite. On obtient 29,51 g de produit attendu. F = 216-218°C.

STADE B : 1-(4-iodophénylcarbamoyl) 2-cyclopropyl 2-oxopropionitrile.

En opérant comme indiqué au stade B de l'exemple 1, en utilisant le 4′-iodo cyanoacétanilide, préparé au stade A précédent, on obtient le produit attendu.

**EXEMPLE 12 : 1-(4-bromo 3-méthylphénylcarbamoyl) 2-cyclopropyl 2-oxopropionitrile.**

STADE A : 4-bromo 3-méthyl cyanoacétanilide.

On dissout 0,457 g d'acide cyanoacétique et 1 g de 4-bromo 3-méthylaniline dans 30 $cm^3$ de chlorure de méthylène, ajoute en 2 minutes, sous agitation à 40 °C, 1,135 g de dicyclohexylcarbodiimide dans 5 $cm^3$ de chlorure de méthylène. La température reste supérieure à 40 °C pendant l'addition ; on agite le mélange 1 heure à température ambiante, filtre la dicyclohexylurée, évapore le solvant, chromatographie le résidu en éluant au chlorure de méthylène comprenant des quantités croissantes d'acétate d'éthyle. On obtient le produit attendu avec un rendement de 87%.

STADE B : 1-(4-bromo 3-méthylphénylcarbamoyl) 2-cyclopropyl 2-oxopropionitrile.

A 300 mg du produit obtenu ci-dessus en solution dans 12 $cm^3$ de tétrahydrofuranne, on ajoute une quantité catalytique d'imidazole, en maintenant l'agitation sous atmosphère d'azote. On ajoute 626 mg d'hydrure de sodium, agite 15 minutes à température ambiante. On ajoute 124 mg de chlorure de cyclopropane carbonyle en 3 minutes et agite 2 heures à température ambiante. On verse le milieu réactionnel sur de l'eau glacée, acidifie à pH = 2 avec de l'acide chlorhydrique (1M), agite 1 minute, filtre le précipité formé, le lave à l'eau puis à l'éther et obtient le produit attendu avec un rendement de 88,3%.

**EXEMPLE 13 : 1-(3,4-méthylènedioxyphénylcarbamoyl) 2-cyclopropyl 2-oxopropionitrile.**

STADE A : 3,4-méthylènedioxy cyanoacétanilide.

On ajoute en 1 minute 279 mg d'acide cyanoacétique à une suspension comprenant 685 mg de pentachlorure de phosphore dans 10 $cm^3$ de chlorure de méthylène sous agitation en maintenant à température ambiante. On chauffe au reflux 30 minutes, agite sous courant d'azote pendant 2 minutes, ajoute 300 mg de 3,4-méthylènedioxyaniline et chauffe 10 minutes au reflux, refroidit à température ambiante, verse sur 10 $cm^3$ d'eau. On agite 30 minutes, filtre, lave à l'eau puis à l'éther et à l'acétate d'éthyle. On obtient 330 mg de produit attendu.

STADE B : 1,(3,4-méthylènedioxyphénylcarbamoyl) 2-cyclopropyl 2-oxopropionitrile.

En opérant comme indiqué au stade B de l'exemple 1, en utilisant le 3,4-méthylènedioxy-cyanoacétanilide, préparé au stade A précédent, on obtient le produit attendu.

En opérant selon les modes opératoires indiqués ci-dessus, à partir des composés appropriés, on a préparé les produits des exemples suivants :

**EXEMPLE 14 : 2-cyano 3-cyclopropyl 3-oxo N-méthyl N-(4-chlorophényl) propionamide.**

**EXEMPLE 15 : 1-(4-chloro 2-méthylphénylcarbamoyl) 2-cyclopropyl 2-oxopropionyitrile.**

**EXEMPLE 16 : 1-(3,4-difluorophénylcarbamoyl) 2-cyclopropyl 2-oxopropionitrile.**

**EXEMPLE 17 : 1-(4-méthoxyphénylcarbamoyl) 2-cyclopropyl 2-oxopropionitrile.**

**EXEMPLE 18 : 1-(4-cyanophénylcarbamoyl) 2-cyclopropyl 2-oxopropionitrile.**

**EXEMPLE 19 : 1-(3,5-dichlorophénylcarbamoyl) 2-cyclopropyl 2-oxopropionitrile.**

**EXEMPLE 20 : 1-(4-chloro 3-méthylphénylcarbamoyl) 2-cyclopropyl 2-oxopropionitrile.**

**EXEMPLE 21 : 1-(3-trifluorométhylphénylcarbamoyl) 2-cyclopropyl 2-oxopropionitrile.**

**EXEMPLE 22 : 1-(4-méthylphénylcarbamoyl) 2-cyclopropyl 2-oxopropionitrile.**

**EXEMPLE 23 : 1-(4-chloro 3-trifluorométhylphénylcarbamoyl) 2-cyclopropyl 2-oxopropionitrile.**

**EXEMPLE 24: 1-(phénylcarbamoyl) 2-cyclopropyl 2-oxopropionitrile.**

**EXEMPLE 25 : 1-(3-méthyl 4-trifluorométhylphénylcarbamoyl) 2-cyclopropyl 2-oxopropionitrile.**

**EXEMPLE 26 : 1-(4-iodo 3-méthylphénylcarbamoyl) 2-cyclopropyl 2-oxopropionitrile.**

**EXEMPLE 27 : 1-(4-fluoro 3-méthylphénylcarbamoyl) 2-cyclopropyl 2-oxopropionitrile.**

**EXEMPLE 28 : 1-(4-cyano 3-méthylphénylcarbamoyl) 2-cyclopropyl 2-oxopropionitrile.**

**EXEMPLE 29 : 1-(4-(2,2,2-trifluoroéthoxy) phénylcarbamoyl) 2-cyclopropyl 2-oxopropionitrile.**

**EXEMPLE 30 : 1-(3-méthyl 4-nitrophénylcarbamoyl) 2-cyclopropyl 2-oxopropionitrile.**

**EXEMPLE 31 : 1-(4-t-butylphénylcarbamoyl) 2-cyclopropyl 2-oxopropionitrile.**

**EXEMPLE 32 : 1-(3-méthylphénylcarbamoyl) 2-cyclopropyl 2-oxopropionitrile.**

**EXEMPLE 33 : 1-(4-trifluorométhylthiophénylcarbamoyl) 2-cyclopropyl 2-oxopropionitrile.**

**EXEMPLE 34 : 1-(4-méthoxycarbanylphénylcarbamoyl) 2-cyclopropyl 2-oxopropionitrile.**

**EXEMPLE 35 : 1-(4-acétylphénylcarbamoyl) 2-cyclopropyl 2-oxopropionitrile.**

**EXEMPLE 36 : 1-(3,4-diméthoxyphénylcarbamoyl) 2-cyclopropyl 2-oxopropionitrile.**

**EXEMPLE 37 : 1-(3-chloro 4-méthylphénylcarbamoyl) 2-cyclopropyl 2-oxopropionitrile.**

**EXEMPLE 38 : 1-(4-méthylthiophénylcarbamoyl) 2-cyclopropyl 2-oxopropionitrile.**

**EXEMPLE 39 : 1-(3-éthyl 4-nitrophénylcarbamoyl) 2-cyclopropyl 2-oxopropionitrile.**

**EXEMPLE 40 : 2-cyano 3-cyclopropyl 3-oxo N-méthyl N-(3-méthyl 4-trifluorométhylphényl) propionamide.**

**EXEMPLE 41 : 1-(4-bromodifluorométhoxy 3-méthylphénylcarbamoyl) 2-cyclopropyl 2-oxopropionitrile.**

**EXEMPLE 42 : 2-cyano 3-cyclopropyl 3-oxo N-méthyl N-(4-cyanophényl) propionamide.**

**EXEMPLE 43 : 2-cyano 3-cyclopropyl 3-oxo N-méthyl N-(4-nitrophényl) propionamide.**

**EXEMPLE 44 : 1-(3-méthyl 4-trifluorométhoxyophénylcarbamoyl) 2-cyclopropyl 2-oxopropionitrile.**

**EXEMPLE 45 : 1-(3-méthyl 4-pentafluoroéthylphénylcarbamoyl) 2-cyclopropyl 2-oxopropionitrile.**

**EXEMPLE 46 : 2-cyano 3-cyclopropyl 3-oxo N-méthyl N-(4-bromo 3-méthylphényl) propionamide.**

**EXEMPLE 47 : 1-(4-chloro 3-éthylphénylcarbamoyl) 2-cyclopropyl 2-oxopropionitrile.**

**EXEMPLE 48 : 1-(4-carboxyphénylcarbamoyl) 2-cyclopropyl 2-oxopropionitrile.**

**EXEMPLE 49 : 1-(4-cyano 3-méthylphénylcarbamoyl) 2-cyclopropyl 2-oxopropionitrile.**

**Préparation du 1-(bromodifluorométhoxy) 2-méthyl 4-aminobenzène utilisé au départ de l'exemple 41.**

On dissout 0,92 g de sodium dans 30 $cm^3$ d'éthanol et ajoute 6 g de 2-méthyl 4-nitrophénol. On évapore le solvant sous pression réduite, puis ajoute du benzène. On introduit ce sel de sodium dans un mélange comprenant 24 $cm^3$ de diméthylformamide, 30 $cm^3$ de dibromodifluorométhane et un peu d'éthanethiol comme catalyseur. On chauffe 10 heures à 70 °C, verse sur de la glace et extrait à l'acétate d'éthyle. On lave avec une solution aqueuse d'hydroxyde de sodium 0,5M puis à l'eau, sèche et évapore le solvant sous pression réduite. Après chromatographie sur silice (éluant : acétate d'éthyle-hexane 4-96), on obtient 0,20 g de 1-(difluorométhoxy) 2-méthyl 4-nitrobenzène et 3,35 g de 1-(bromodifluorométhoxy) 2-méthyl 4-aminobenzène que l'on hydrogène en présence de palladium sur charbon actif et obtient le produit attendu.

**Préparation du 2-méthyl 4-amino 1-trifluorométhoxybenzène utilisé au départ de l'exemple 44.**

On chauffe 4 heures à 175 °C dans un flacon scellé, 0,3 g de 1-(bromodifluorométhoxy) 2-méthyl 4-nitrobenzène, 0,120 g de trifluorure d'antimoine et 0,02 g de pentaclhorure d'antimoine comme catalyseur. On dilue le mélange avec de l'éther, lave à l'eau, sèche et évapore le solvant sous pression réduite ; on obtient 0,13 g de 2-méthyl 4-nitro 1-trifluorométhoxybenzène que l'on hydrogène en présence de palladium sur charbon actif et obtient le produit attendu.

**Préparation du 2-méthyl 4-amino 1-pentafluoroéthylbenzène utilisé au départ de l'exemple 45.**

On mélange sous argon dans une enceinte scellée 2,36 g de 1-iodo 2-méthyl 4-nitrobenzène et 2,2 g de cuivre en poudre (Org. Synthesis Coll. Vol II (1948) 445, lavé à l'eau, à l'acétone et séché sous pression réduite) dans 10 $cm^3$ de diméthylformamide. On refroidit à -60 °C et ajoute 11,5 g d'iodure de pentafluoroéthyle. On agite à 160 °C sous une pression de 3,5 bars pendant 16 heures, refroidit dans la glace et ramène à l'ambiance. On verse le mélange sur de la glace et extrait à l'acétate d'éthyle. On lave à l'eau, sèche et évapore le solvant sous pression réduite. Après chromatographie sur silice (éluant : pentane à 2-3% de dichlorométhane), on obtient 1,5 g de 2-méthyl 4-nitro 1-pentafluoroéthylbenzène que l'on hydrogène en présence de palladium et obtient le produit attendu.

Les analyses spectrométriques, les résultats de la microanalyse, les rendements et les points de fusion sont donnés dans les tableaux ci-après.

## TABLEAU I

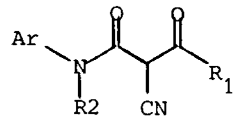

| Exemple | Ar | R2 | R1 | Rendt | F°C | Spectre IR cm-1 |
|---|---|---|---|---|---|---|
| 1 | 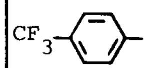 | H | 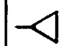 | 70% | 212-3 | 3280(NH), 2202(CN), 1620, 1602, 1575, 1540, 1410, 1320, 1100 |
| 2 | 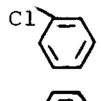 | H | 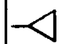 | 44% | 137-9 | 3280(NH), 2203(CN), 1620, 1600, 1568, 1535, 1477, 1408, 1395, 1345, 1300, 1253, 1221 |
| 3 | 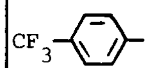 | H | 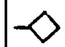 | 66% | 177-8 | 3260(NH), 2200(CN), 1621, 1601, 1574, 1540, 1317, 1150, 1110, 1056, 832 |
| 4 | 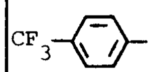 | H | 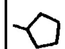 | 73% | 177-80 | 3280(NH), 2000(CN), 1625, 1600, 1574, 1540, 1320, 1118, 1108, 1061, 832 |
| 5 | 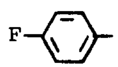 | H | 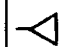 | 74% | 187-8 | 3310(NH), 2200(CN), 1610, 1580, 1540, 1526, 1502, 1403, 1200, 885, 823 |
| 6 | 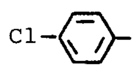 | H | 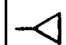 | 89% | 189-91 | 3280(NH), 2200(CN), 1620, 1597, 1569, 1539, 1525, 1482, 1381, 1392, 1302, 1230, 885, 820 |
| 7 | 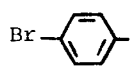 | H | 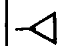 | 81% | 190-2 | 3275(NH), 2200(CN), 1620, 1590, 1565, 1540, 1520, 1479, 1388, 1343, 1302, 1229, 886 |
| 8 | 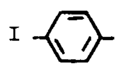 | H | 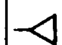 | 81% | 182-4 | 3275(NH), 2204, 1615, 1595, 1575, 1540, 1478, 1404, 1387, 1348, 886, 811 |
| 9 | 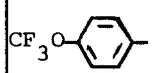 | H | 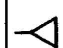 | 83% | 173-5 | 3285(NH), 2206(CN), 1627, 1608, 1579, 1540, 1502, 1417, 1381, 1270, 1233, 1210, 1153, 891, 842 |
| 10 | 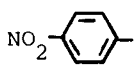 | H | 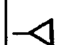 | 94% | 235-6 | 3290(NH), 2205(CN),1612,1555,1496,1418,1338,1310, 1270, 1242, 1190, 1180, 1114, 1084, 1062, 890, 864 |

**TABLEAU I (suite)**

| Exemple | Spectre RMN | Formule PM | C% Calculé Trouvé | H% | N% | X% |
|---|---|---|---|---|---|---|
| 1 | CDCl3 - 15,64(1H,s); 7,77(1H,s); 7,64(4H,s); 2,16(1H,m); 1,37(2H,m); 1,18(2H,m) | C14H1102N2F3<br>282,26 | 56,76<br>56,81 | 3,74<br>3,79 | 9,46<br>9,45 | 19,25<br>19,25 |
| 2 | CDCl3 - 1570(1H,s); 7,65(1H,s); 7,63(1H,s); 7,25(3H,m);2,16(1H,m); 1,34(2H,m);1,18(2H,m) | C13H11N2O2Cl<br>262,69 | 59,44<br>59,41 | 4,22<br>4,28 | 10,66<br>10,65 | 13,49<br>13,52 |
| 3 | CDCl3 - 15,61(1H,s); 7,76(1H,s); 7,64(4H,s); 3,66(1H,m); 2,24(6H,m) | C15H13N2O2F3<br>310,28 | 58,07<br>58,03 | 4,22<br>4,28 | 9,02<br>9,02 | 18,37<br>18,43 |
| 4 | CDCl3 - 15,51(1H,s); 7,72(1H,s); 7,64(4H,s); 3,22(1H,m); 1,85(8H,m) | C16H15N2O3F3<br>324,31 | 59,26<br>59,17 | 4,66<br>4,69 | 8,64<br>8,63 | 17,58<br>17,63 |
| 5 | CDCl3 - 15,83(1H,s); 7,61(1H,s); 7,42(2H,m); 7,06(2H,m); 2,15(1H,m);1,32(2H,m);1,16(2H,m) | C13H11N2O2F<br>246,24 | 63,41<br>- | 4,50<br>- | 11,38<br>- | 13,00<br>- |
| 6 | CDCl3 - 15,77(1H,s); 7,63(1H,s); 7,44(2H,d); 7,33(2H,d); 2,14(1H,m);1,31(2H,m);1,16(2H,m) | C13H11N2O2Cl<br>262,69 | 59,44<br>59,35 | 4,22<br>4,30 | 10,66<br>10,67 | 13,49<br>13,51 |
| 7 | CDCl3 - 15,76(1H,s); 7,62(1H,s); 7,48(2H,d); 7,38(2H,d); 2,14(1H,m);1,32(2H,m);1,25(2H,m) | C13H11N2O2Br<br>307,15 | 50,84<br>- | 3,61<br>- | 9,12<br>- | 26,02<br>- |
| 8 | CDCl3 - 15,71(1H,s); 7,67(2H,m); 7,53(1H,s); 7,26(2H,m); 2,14(1H,m);1,34(2H,m);1,16(2H,m) | C13H11N2O2I<br>354,14 | 44,09<br>- | 3,13<br>- | 7,91<br>- | 35,83<br>- |
| 9 | CDCl3 - 15,76(1H,s); 7,73(1H,s); 7,53(2H,d); 7,22(2H,d); 2,15(1H,m);1,31(2H,m);1,18(2H,m) | C14H11N2O3F3<br>312,26 | 53,85<br>- | 3,55<br>- | 8,97<br>- | 18,25<br>- |
| 10 | DMSO - 12,27(1H,s); 8,19(2H,d); 7,79(2H,d); 2,20(1H,m); 0,89(4H,m) | C13H11N3O4<br>273,25 | 57,14,<br>- | 4,07<br>- | 15,38<br>- | |

**TABLEAU I (suite)**

| Exemple | Ar | R2 | R1 | Rendt | F°C | Spectre IR cm-1 |
|---|---|---|---|---|---|---|
| 11 | 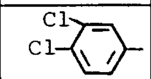 | H | 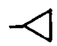 | 60% | 196 | 3305(NH), 2205(CN), 1630, 1602, 1570, 1525, 1480, 1450, 1405, 1342, 1302, 1270, 1250, 1225 |
| 12 | 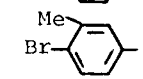 | H | 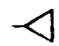 | 88% | 158 | 3280(NH), 2180(CN), 1600, 1580, 1510, 1470, 1435, 1395, 1330, 1290, 1270, 1245, 1220 |
| 13 | 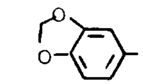 | H | 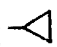 | 32% | 152 | 3305(NH), 3075, 2885, 2195(CN), 1630, 1580, 1540, 1480, 1425, 1280, 1255, 1235, 1200 |
| 14 | 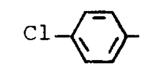 | CH3 | 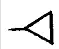 | 45% | 135-7 | 2180(CN), 1591, 1581, 1562, 1550, 1467, 1425, 1389, 1071, 1042, 1017, 877 |
| 15 | 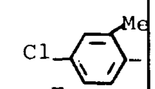 | H | 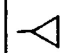 | 45% | 167 | 3290(NH),3020,2205(CN),1890,1400,1350,1295,1185, 1120,1080,1060,1025,985,940,885,850,805,770,750 |
| 16 | 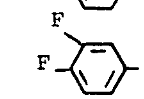 | H | 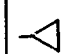 | 50% | 177 | 3301(NH),2210(CN),1602,1545,1510,1440,1345,1278,1240 1208,1150,1110,1080,1065,1025,965,890,805,775,665 |
| 17 | 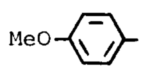 | H | 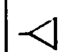 | | | |
| 18 | 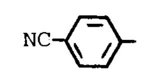 | H | 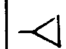 | 53% | 236,5 | 3350(NH), 2180(CN), 1890(CN) |
| 19 | 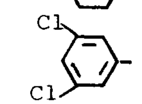 | H | 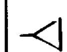 | 72% | 159,5 | |
| 20 | 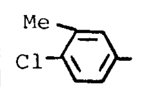 | H | 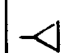 | | | |

**TABLEAU I (suite)**

| Exemple | Spectre RMN | Formule PM | C% Calculé Trouvé | H% | N% | X% |
|---|---|---|---|---|---|---|
| 11 | DMSO - 11,536(1H,s); 8,04(1H,m); 7,52(1H,m); 7,45(1H,m); 2,19(1H,m); 0,95(4H,m) | $C_{13}H_{10}Cl_2N_2O_2$<br>297,14 | 52,55<br>52,37 | 3,39<br>3,45 | 9,43<br>9,37 | 23,86<br>- |
| 12 | DMSO - 10,97(1H,s); 7,54(2H,m); 7,39(1H,m); 2,34(3H,s);2,19(1H,m); 1,03(4H,m) | $C_{14}H_{13}N_2O_2Br$<br>321,164 | 52,35<br>52,37 | 4,08<br>4,10 | 8,72<br>8,65 | 24,88<br>24,10 |
| 13 | $CDCl_3$ - 15,89 (1H,s); 7,44(1H,s);7,10(1H,m); 6,78(2H,m); 5,99(2H,s);2,10(1H,m);1,26(4H,m) | $C_{14}H_{12}N_2O_4$<br>272,266 | 61,76<br>61,65 | 4,44<br>4,47 | 10,29<br>10,18 | -<br>- |
| 14 | $CDCl_3$ - 17,09(1H,s); 7,44(2H,d); 7,23(2H,d); 3,34(3H,s); 2,13(1H,m);1,24(2H,m);1,03(2H,m) | $C_{14}H_{13}N_2O_2Cl$<br>276,72 | 60,77<br>- | 4,73<br>- | 10,12<br>- | 12,82<br>- |
| 15 | DMSO - 10,807(1H,s); 7,72(1H,m); 7,35(1H,m); 7,27(1H,m); 2,22(3H,s);2,19(1H,m);1,02(4H,m) | $C_{14}H_{13}N_2O_2Cl$<br>276,714 | 60,76<br>60,69 | 4,74<br>4,77 | 10,13<br>10,10 | 12,81<br>12,82 |
| 16 | DMSO - 11,20(1H,s); 7,80(1H,m); 7,37(2H,m); 2,19(1H,m); 1,01(4H,m) | $C_{13}H_{10}N_2O_2F_2$<br>264,23 | 59,09<br>- | 3,81<br>- | 10,60<br>- | 14,38<br>- |
| 17 | | | | | | |
| 18 | DMSO - 11,89(1H,s); 7,74(4H,m); 2,2 (1H,m); 0,9 (4H,m) | $C_{14}H_{11}N_3O_2$<br>253,932 | 66,21<br>- | 4,37<br>- | 16,55<br>- | -<br>- |
| 19 | DMSO - 11,89(1H,s); 7,66(2H,s); 7,18(1H,s); 2,19(1H,m); 0,88(4H,m) | $C_{13}H_{10}N_2Cl_2$<br>297,132 | 52,55<br>- | 3,39<br>- | 9,43<br>- | 23,86<br>- |
| 20 | | $C_{14}H_{14}N_2O_2$<br>242,2 | 69,4<br>- | 5,825<br>- | 11,57<br>- | 13,21 |

**TABLEAU I (suite)**

| Exemple | Ar | R2 | R1 | F°C | Spectre IR cm-1 |
|---|---|---|---|---|---|
| 21 | 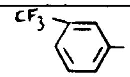 | H | 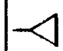 | - | 3710(NH), 2035(CN), 1545, 1520, 1495, 1460, 1400, 1310, 1260, 1240, 1170, 1130, 1060, 1025 |
| 22 | 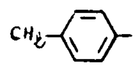 | H | 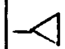 | - | |
| 23 | 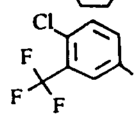 | H | 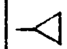 | 207 | 3280(NH), 2220(CN), 1620, 1520, 1470, 1400, 1340, 1305, 1260, 1220, 1080, 1020, 975,910,890,820, 755, 685, 655, 620 |
| 24 | 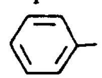 | H | 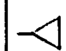 | 117 | 3290(NH), 2220(CN), 1600, 1450, 1415, 1350, 1320, 1265, 1245,1195,1180,1090,1065,1030,990,920,895,805,770,755,695 |
| 25 | 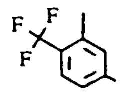 | H | 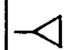 | 186-8 | 3395(NH), 2204(CN), 1630, 1600, 1580, 1545, 1409, 1346, 1310, 1156, 1110, 1097, 1037, 1027, 888, 877, 831 |
| 26 | 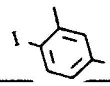 | H | 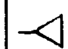 | 163-5 | 3284(NH), 2000(CN), 1623, 1599, 1565, 1525, 1469, 1400, 1371, 1338, 1301, 1250, 1238, 882 |
| 27 | 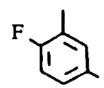 | H | 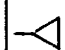 | 140-2 | 3280(NH), 2002(CN), 1620, 1573, 1560, 1540, 1491, 1341, 1204, 886 |
| 28 | 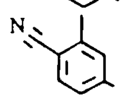 | H | 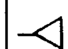 | 300 | 3280(NH), 2195s(CN), 2145w(CN), 1630, 1588, 1551, 1500, 1410, 1332, 1309, 1254, 1230, 877, 821 |
| 29 | 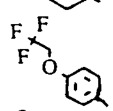 | H | 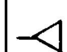 | - | 3380(NH), 2002(CN), 1621, 1602, 1578, 1551, 1540, 1502, 1477, 1350, 1280, 1220, 1150, 1071, 889 |
| 30 | 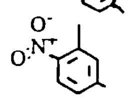 | H | 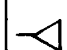 | 223-6 | 3300, 3120, 2930, 2220, 1630, 1570, 1550, 1500, 1450, 1420, 1380, 1350, 1290, 1270, 1240, 1090, 1070, 1040 |

**TABLEAU I (suite)**

| Exemple | Spectre RMN | Formule PM | C% H% N% X%<br>Calculé<br>Trouvé | | | |
|---|---|---|---|---|---|---|
| 21 | DMSO - 11,57(1H,s); 8,12(1H,s); 7,68(1H,m); 7,36(1H,m); 2,20(1H,m); 0,91(4H,m) | C14H11N2O2F3<br>296,24 | 56,76<br>- | 3,74<br>- | 9,46<br>- | 19,25<br>- |
| 22 | | C14H14N2O2<br>242,26 | 69,4<br>- | 5,82<br>- | 11,86<br>- | -<br>- |
| 23 | DMSO - 11,49(1H,s); 8,23(1H,m); 7,81(1H,m); 7,67(1H,m); 2,22(1H,m); 1,07(4H,m) | C14H10ClF3N2O2<br>330,70 | 50,85<br>50,71<br>Calculé F% 17,23 | 3,05<br>3,16 | 8,47<br>8,39 | 10,72<br>10,73 |
| 24 | DMSO - 10,68(1H,s); 7,53(2H,m); 7,36(2H,m); 7,17(1H,m); 2,20(1H,m);1,09(4H,m) | C13H12N2O2<br>228,25 | 68,41<br>68,40 | 5,30<br>5,37 | 12,27<br>12,32 | -<br>- |
| 25 | CDCl3 - 15,63(1H,s); 7,59(2H,d); 7,43(2H,d); 2,49(3H,s); 2,15(1H,m);1,35(2H,m);1,19(2H,m) | C15H13F3N2O2<br>310,28 | 58,07<br>58,00 | 4,22<br>4,30 | 9,03<br>9,04 | 18,37<br>18,27 |
| 26 | CDCl3 - 15,73(1H,s); 7,77(1H,d); 7,45(1H,s); 7,37(1H,d); 7,06(1H,dd); 2,43(3H,s); 2,14(1H,m); 1,34(2H,m); 1,17(2H,m) | C14H13IN2O2<br>368,18 | 45,67<br>- | 3,56<br>- | 7,61<br>- | 34,47<br>- |
| 27 | CDCl3 - 15,85(1H,s); 7,49(1H,s); 7,24(2H,m); 7,00(1H,t);2,28(3H,s); 1,33(2H,m);1,16(2H,m) | C14H13FN2O2<br>260,27 | 64,61<br>- | 5,03<br>- | 10,76<br>- | 7,30<br>- |
| 28 | DMSO - 12,01(1H,s); 7,63(3H,m); 2,45(3H,s); 2,21(1H,m); 0,88(4H,m) | C15H13N3O2<br>267,29 | 67,41<br>- | 4,90<br>- | 15,72<br>- | -<br>- |
| 29 | CDCl3 - 15,87(1H,s); 7,52(2H,m); 7,42(2H,m); 7,95(2H,m);4,34(2H,q);2,15(1H,m);1,33(2H,m); 1,16(2H,m) | C15H13F3N2O3<br>326,28 | 55,22<br>- | 4,02<br>- | 8,59<br>- | 17,47<br>- |
| 30 | DMSO - 11,65(1H,s); 8,02(1H,d); 7,65(2H,m); 2,53(3H,s); 2,17(1H,m); 0,95(4H,m) | C14H13N3O4<br>287,28 | 58,53<br>- | 4,56<br>- | 14,63<br>- | -<br>- |

**TABLEAU I (suite)**

| Exemple | Ar | R2 | R1 | F°C | Spectre IR cm-1 |
|---|---|---|---|---|---|
| 31 | 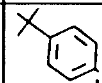 | H | 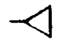 | 141-3 | 3260, 2970, 2210, 1910, 1590, 1410, 1350, 1300, 1270, 1195, 1120,1095,1070,1020,995,950,905,870,840,825,805,770,730,675 |
| 32 | 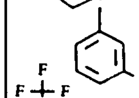 | H | 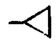 | 102-4 | |
| 33 | 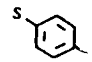 | H | 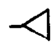 | 175-6 | 3280(NH), 2212(CN), 1621, 1597, 1570, 1527, 1490, 1346, 1310, 1130(s), 1110, 1090, 892, 829 |
| 34 | 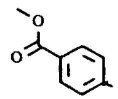 | H | 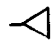 | 188-9 | 3360(m),3322(m),2200(m),1710(s),1576(s),1524(s),1429(m), 1409(s),1348(m),1318(m),1275(s),1241(m),1189(m),1107(m), 1081(m),985(m),894(m),763(m) |
| 35 | 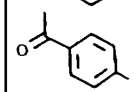 | H | 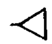 | 158-60 | 3300, 2920, 2840, 2200, 1660, 1580, 1510, 1450, 1345, 1305, 1260,1240,1175,1065,1035,980,915,890,870,835,820,805,760 |
| 36 | 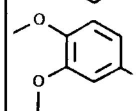 | H | 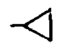 | 162-4 | 3290,2905,2820,2210,1575,1550,1505,1460,1435,1410,1395, 1355,1290,1270,1250,1225,1160,1130,1080,1020,890,840,805, 760,710,690,680 |
| 37 | 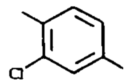 | H | 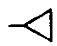 | 122-4 | 3290, 2200, 1565, 1520, 1490, 1440, 1405, 1380, 1340, 1305, 1250, 1230, 1180, 1080, 1040, 995, 885, 865, 810, 680 |
| 38 | 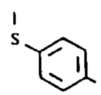 | H | 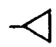 | 141-2 | 3280, 2205, 1570, 1525, 1485, 1395, 1360, 1305, 1280, 1235, 1080, 1060, 970, 890, 815, 760, 665 |
| 39 | 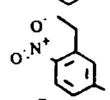 | H | 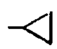 | 179-81 | 3290(s), 2210(s), 1610(m), 1570(s), 1525(s), 1410(s), 1330(s), 1240(s), 880(m) |
| 40 | 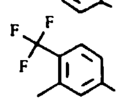 | CH3 | 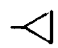 | 108,5-110 | 2220, 1560, 1460, 1380, 1320, 1170, 1130, 1050 |

## TABLEAU I (suite)

| Exemple | Spectre RMN | Formule PM | C% Calculé Trouvé | H% | N% | X% |
|---|---|---|---|---|---|---|
| 31 | DMSO - 10,65(1H,s); 7,37(2H,m); 7,26(1H,m); 6,97(1H,m); 2,32(3H,s);2,19(1H,m);1,08(4H,m) | C17H20N2O2<br>284,36 | 71,81<br>- | 7,09<br>- | 9,85<br>- | -<br>- |
| 32 | | C14H14N2O2<br>242,28 | 69,41<br>- | 5,82<br>- | 11,56<br>- | -<br>- |
| 33 | CDCl3 - 15,62(1H,s); 7,66(2H,d); 7,58(2H,d); 2,16(1H,m); 1,35(2H,m); 1,19(2H,m); 192,51; 167,94; 138,71; 137,41; 129,39(q,J=309Hz); 121,41; 120,40; 15,91; 11,19 | C14H11F3N2O2S<br>328,32 | 51,22<br>-<br>Calculé S% 9,77 | 3,38<br>- | 8,53<br>- | 17,36<br>- |
| 34 | CDCl3 - 1,14-1,25(2H,m); 1,31-1,41(2H,m); 2,10-2,20(1H,m); 3,92(3H,s); 7,59(2H,d,J=8,6 Hz);7,66(1H,s);8,05(2H,d,J=8,4); 15,63(1H,s) | C15H14N2O4<br>286,29 | 62,93<br>- | 4,93<br>- | 9,78<br>- | -<br>- |
| 35 | DMSO - 11,86(1H,s); 7,92(2H,m); 7,69(2H,m); 2,52(3H,m); 2,17(1H,m); 0,88(4H,m) | C15H14N2O3<br>270,29 | 66,66<br>- | 5,22<br>- | 10,36<br>- | -<br>- |
| 36 | DMSO - 10,44(1H,s); 7,22(1H,m); 7,11(1H,m); 6,95(1H,m); 3,76(6H,s);2,19(1H,m);1,07(4H,m) | C15H16N2O4<br>288,31 | 62,49<br>- | 5,59<br>- | 9,72<br>- | -<br>- |
| 37 | DMSO - 11,21(1H,s); 7,81(1H,s); 7,28(2H,s); 2,30(3H,s); 2,19(1H,m); 0,98(1H,m) | C14H13ClN2O2<br>276,72 | 60,77<br>- | 4,74<br>- | 10,12<br>- | 12,81<br>- |
| 38 | DMSO - 10,66(1H,s); 7,52(2H,m); 7,27(2H,m); 2,52(3H,s); 2,20(1H,m); 1,06(4H,m) | C14H14N2O2S<br>274,34 | 61,29<br>- | 5,14<br>- | 10,21<br>- | 11,69<br>- |

**TABLEAU I (suite)**

| Exemple | Spectre RMN | Formule PM | C% Calculé Trouvé | H% | N% | X% |
|---|---|---|---|---|---|---|
| 39 | DMSO - 11,82(1H,s-NH);8,00(1H,d,J=9Hz,H-5); 7,71(1H,dd,J=9Hz,J=2Hz,H-6);7,63(1H,d,J=2Hz, H-2);2,91(2H,q,J=7,4Hz,éthyl-CH2);2,28-2,16 (1H,m,cyclopropyle 1-H);1,24(3H,t,J=7,4Hz, -CH3);0,97-0,87(4H,m,cyclopropyle) | C15H15N3O4<br>301,30 | 59,80<br>- | 5,02<br>- | 13,95<br>- | -<br>- |
| 40 | CDCl3 - 16,99(1H,s); 7,69(1H,d); 7,23(2H,d); 3,37(3H,s); 2,53(3H,s);2,14(1H,m);1,15(4H,m) | C16H15F3N2O2<br>324,31 | 59,26<br>- | 4,66<br>- | 8,64<br>- | 17,57<br>- |

**TABLEAU I (suite)**

| Exemple | Ar | R2 | R1 | F°C | Spectre IR cm-1 |
|---|---|---|---|---|---|
| 41 | 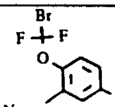 | H | 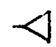 | 165-7 | 3280(NH), 2198(CN), 1620, 1600, 1575, 1552, 1540, 1481, 1400, 1253, 1200, 1185, 1131, 990, 886 |
| 42 | 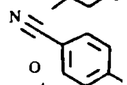 | CH3 | 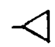 | 141-3 | 2260, 2230, 1570, 1520, 1470, 1390, 1210, 1110, 1080, 1050, 1030 |
| 43 | 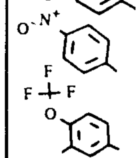 | CH3 | 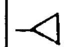 | 141,5-142,5 | 2220, 1670, 1530, 1510, 1480, 1450, 1200, 1100, 1040, 1020 |
| 44 | | H | 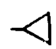 | 148-50 | 3285(NH), 2006(CN), 1627, 1580, 1560, 1543, 1421, 1280, 1260, 1242, 1200, 1141, 889 |
| 45 | 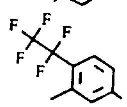 | H | 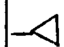 | 135-7 | 3275(NH),2204(CN),1610,1590,1560,1525,1410,1340,1307,1285, 1254, 1185, 1132, 1112, 1066, 962, 881 |
| 46 | 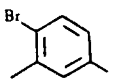 | CH3 | 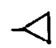 | 138-9 | 2220, 1570, 1490, 1400, 1240, 1210, 1110, 1050 |
| 47 | 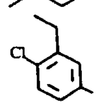 | H | 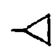 | 169-70 | 3340, 2240, 1635, 1540, 1495, 1320, 900 |
| 48 | 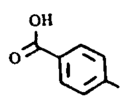 | H | 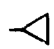 | | |

**TABLEAU I (suite)**

| Exemple | Spectre RMN | Formule PM | C% Calculé Trouvé | H% | N% | X% |
|---|---|---|---|---|---|---|
| 41 | CDCl3 - 15,72(1H,s); 7,54(1H,s); 7,33(3H,m); 2,32(3H,s); 2,15(1H,m);1,33(2H,m);1,17(2H,m) | C15H13BrF2N2O3<br>387,19 | 46,53<br>-<br>Calculé F% 9,81 | 3,38<br>- | 7,24<br>- | 20,64<br>- |
| 42 | CDCl3 - 16,75(1H,s); 7,76(2H,d); 7,37(2H,d); 3,39(3H,s); 2,10(1H,m); 1,18(4H,m) | C15H13N3O2<br>267,29 | 67,41<br>- | 4,90<br>- | 15,72<br>- | -<br>- |
| 43 | CDCl3 - 16,69(1H,s); 8,33(2H,d); 7,45(2H,d); 3,42(3H,s); 2,10(1H,pentet); 1,18(4H,m) | C14H13N3O4<br>287,28 | 58,53<br>- | 4,56<br>- | 14,63<br>- | -<br>- |
| 44 | CDCl3 - 15,70(1H,s); 7,48(1H,s); 7,33(4H,m); 2,32(3H,s);2,12(3H,s);1,31(2H,m);1,17(2H,m) | C15H13F3N2O3<br>326,28 | 55,22<br>- | 4,02<br>- | 8,59<br>- | 17,47<br>- |
| 45 | CDCl3 - 15,62(1H,s);7,62(1H,s);7,46(3H,m); 2,49(3H,t,J=3Hz);2,13(1H,m);1,34(2H,m); 1,19(2H,m) | C16H13F5N2O2<br>360,29 | 53,34<br>- | 3,64<br>- | 7,78<br>- | 26,37<br>- |
| 46 | CDCl3 - 17,16(1H,s); 7,60(1H,d); 7,13(1H,d); 6,96(1H,dd); 3,30(3H,s); 2,44(3H,s); 2,13(1H,pentet); 1,13(4H,m) | C15H15BrN2O2<br>335,21 | 53,75<br>- | 4,51<br>- | 8,36<br>- | 23,84<br>- |
| 47 | CDCl3 - 15,82(1H,s,OH); 7,57(1H,s,-NH); 7,31-7,26(3H,m,aromatique); 2,75(2H,q,J=6Hz, éthyle -CH2); 2,18-2,08(1H,m,cyclopropyle 1-H); 1,36-1,10(4H,m,cyclopropyle); 1,24 (3H,t,J=7,6Hz,éthyle -CH3) | C15H15ClN2O2<br>290,75 | 61,97<br>- | 5,20<br>- | 9,63<br>- | 12,19<br>- |
| 48 | | C14H12N2O4<br>272,26 | 61,76<br>- | 4,44<br>- | 10,29<br>- | -<br>- |

**EXEMPLE 50** :

On a préparé des comprimés répondant à la formule suivante :

| | |
|---|---|
| - Composé de l'exemple 1<br>- Excipient q.s.p. un comprimé terminé à | 20 mg<br>150 mg |
| (Détail de l'excipient : lactose, amidon, talc, stéarate de magnésium). | |

**EXEMPLE 51** :

On a préparé des comprimés répondant à la formule suivante :

| | |
|---|---|
| - Composé de l'exemple 2<br>- Excipient q.s.p. un comprimé terminé à | 20 mg<br>150 mg |
| (Détail de l'excipient : lactose, amidon, talc, stéarate de magnésium). | |

**ACTIVITE PHARMACOLOGIOUE**

Méthodes de tests biochimiques.

**1er test**

Oedème de la patte de rat (PO-R) induit par de la carragénine.

Une heure après l'administration orale des composés du test ou du véhicule témoin à des groupes de rats (n = 6-12, CFHB mâles, d'un poids entre 160-180 g), on injecte 1 mg de carragénine dissoute dans 0,2 ml de solution saline dans le coussinet digital de la patte arrière droite. Les pattes contralatérales reçoivent les injections témoin de solution saline. Les réactions d'oedèmes des pattes sont évaluées trois heures plus tard.

**2ème test**

Oedème à hypersensibilité de type retard de la patte de souris (DTH-M).

Des groupes de souris (n = 8-10), mâles CD-1, d'un poids entre 25-30 g) sont sensibilisés par une injection sous-cutanée de 1 mg de sérum-albumine bovine méthylé (MBSA) dans des volumes de 0,2 ml d'une solution saline/émulsion d'adjuvant complet de Freund (FCA). Des groupes témoin négatifs reçoivent des injections de solution saline/émulsion de FCA. Les réactions DHT d'oedème de la patte sont évaluées 24 heures après le défi dans le coussinet digital de la patte arrière droite avec 0,1 mg de MBSA dans des volumes de 0,05 ml de solution saline le 7ème jour après la sensibilisation. Les pattes contralatérales reçoivent des injections témoin de solution saline. Les composés du test ou les véhivules témoin sont administrés oralement une fois par jour les 4ème, 5ème et 6ème jours et deux fois par jour le 7ème jour, 1 heure avant et 6 heures après le défi au MBSA.

**3ème test**

Oedème à hypersensibilité de type retard de la patte de rat (DTH-R).

Des groupes de rats (n = 8-12, mâles CFHB, d'un poids entre 160-180 g) sont sensibilisés par une injection sous-cutanée à la base de la queue avec des volumes de 0,1 ml de FCA. Des groupes témoin négatifs reçoivent une injection d'adjuvant incomplet de Freund. Les réactions DTH d'oedème de la patte sont évaluées 24 heures après le défi dans le coussinet digital de la patte arrière droite avec 0,4 mg d'un extrait antigène de Mycobacterium tuberculosis dans des volumes de 0,2 ml de solution saline le 7ème jour après la sensibilisation. Les pattes contralatérales reçoivent des injections témoin de solution saline.

Les composés du test sont administrés oralement une fois par jour les 4ème, 5ème et 6ème jour et deux fois par jour le 7ème jour, 1 heure avant et 6 heures après le défi antigénique.

Les résultats de ces tests sont donnés au tableau II.
Les doses sont données en unités de mg/kg p.o.

**TABLEAU II**

| Exemple | Test 1 | | Test 2 | | Test 3 | |
|---|---|---|---|---|---|---|
| | % inhibition | Dose | % inhibition | Dose | % inhibition | Dose |
| 1 | 17 | (50) | 85 | (30) | 53 | (10) |
| 2 | 63 | (50) | 55 | (100) | 25 | (50) |
| 3 | 43 | (50) | 4 | (100) | -1 | (50) |
| 4 | 31 | (50) | 58 | (100) | 19 | (50) |
| 5 | 44 | (50) | 79 | (30) | 46 | (10) |
| 6 | 12 | (50) | 76 | (30) | 74 | (3) |
| 7 | 32 | (50) | 64 | (30) | 61 | (3) |
| 8 | 17 | (50) | 69 | (30) | 53 | (3) |
| 9 | 39 | (50) | 85 | (30) | 104 | (3) |
| 10 | 32 | (50) | 64 | (30) | 30 | (10) |
| 11 | 35 | (50) | 86 | (100) | 66 | (50) |
| 12 | 48 | (50) | 47 | (30) | 39 | (10) |
| 13 | 31 | (50) | 79 | (100) | 22 | (10) |

**TABLEAU II (suite)**

| Exemple | Test 1 | | Test 2 | | Test 3 | |
|---|---|---|---|---|---|---|
| | % inhibition | Dose | % inhibition | Dose | % inhibition | Dose |
| 14 | -26 | (50) | 74 | (100) | 49 | (10) |
| 15 | 34 | (50) | 54 | (30) | 67 | (50) |
| 16 | 46 | (50) | 87 | (30) | 68 | (10) |
| 18 | 26 | (50) | 73 | (30) | 47 | (10) |
| 19 | 24 | (50) | 37 | (30) | 64 | (50) |
| 20 | 31 | (50) | 41 | (30) | 42 | (10) |
| 25 | 38 | (50) | 81 | (30) | 59 | (10) |
| 26 | -4 | (50) | 58 | (100) | 30 | (10) |
| 27 | 32 | (50) | 62 | (30) | 41 | (10) |
| 28 | 2 | (50) | 66 | (30) | 50 | (10) |
| 30 | 8 | (50) | 47 | (10) | 23 | (30) |
| 33 | 7 | (50) | 90 | (3) | 39 | (30) |

## Revendications

**Revendications pour les Etats contractants suivants : AT, BE, CH, DE, DK, FR, GB, IT, LI, LU, NL, SE**

**1.** Nouveaux 3-cycloalkyl-propanamides répondant à la formule générale (I) :

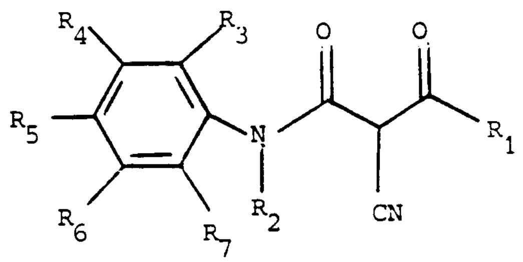

(I)

dans laquelle :

- $R_1$ représente un groupement cycloalkyle renfermant de 3 à 6 atomes de carbone,
- $R_2$ représente un atome d'hydrogène, un radical alkyle renfermant de 1 à 3 atomes de carbone,
- $R_3$, $R_4$, $R_5$, $R_6$ et $R_7$, identiques ou différents, représentent un atome d'hydrogène, un atome d'halogène, un radical alkyle linéaire ou ramifié renfermant de 1 à 6 atomes de carbone, un radical alcoxy linéaire ou ramifié renfermant de 1 à 6 atomes de carbone, un radical alkylthio renfermant de 1 à 6 atomes de carbone, un radical $-(CH_2)_m-CF_3$, $-O-(CH_2)_m-CF_3$, $-S-(CH_2)_m-CF_3$ dans lesquels m représente un nombre entier compris entre 0 et 3, un radical $-CF_2-Hal$, $-OCF_2-Hal$,

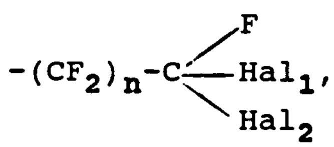

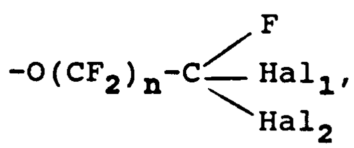

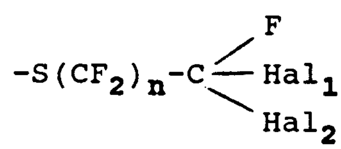

ou $O(CF_2)_n-CF-Hal_3-CF_3$, dans lequel n représente un nombre entier compris entre 1 et 3, $Hal_1$ et $Hal_2$ identiques ou différents, Hal et $Hal_3$ représentent un atome d'halogène,

ou $R_3$, $R_4$, $R_5$, $R_6$ et $R_7$ identiques ou différents représentent un groupement nitro, un groupement azido, un groupement nitrile, un groupement -CO-R' dans lequel R' représente un radical hydroxy, alkyle ou alcoxy renfermant de 1 à 3 atomes de carbone ou, $R_4$ et $R_5$ forment ensemble un groupement $-O-CH_2-O-$, leurs formes tautomères, ainsi que leurs sels d'addition avec les bases minérales ou organiques.

**2.** Nouveaux 3-cycloalkyl-propanamides tels que définis par la formule (I) de la revendication 1, caractérisés en ce que dans ladite formule (I), $R_3$, $R_4$, $R_5$, $R_6$ et $R_7$ identiques ou différents, représentent un atome d'hydrogène, un atome de fluor ,de chlore, de brome ou d'iode, un radical méthyle, éthyle, terbutyle, méthoxy, méthylthio, trifluorométhyle, trifluorométhoxy, trifluorométhylthio, pentafluoroéthyle, bromodifluorométhoxy, acétyle, hydroxycarbonyle, méthoxycarbonyle, nitro, azido, nitrile ou $R_4$ ou $R_5$ forment ensemble un groupement $O-CH_2-O-$, $R_2$ représente un atome d'hydrogène, ou un radical méthyle, $R_1$ a la signification déjà indiquée, ainsi que leurs sels d'addition avec les bases minérales ou organiques.

3. Nouveaux 3-cycloalkyl-propanamides répondant à la formule (I) selon la revendication 1 ou 2, caractérisés en ce que dans ladite formule (I), $R_1$ représente un groupement cyclopropyle, $R_2$ représente un atome d'hydrogène ou un radical méthyle, $R_3$, $R_4$, $R_5$, $R_6$ et $R_7$ identiques ou différents, représentent un atome d'hydrogène, un atome de fluor, de chlore ou d'iode, un radical méthyle, trifluorométhyle ou nitro, ainsi que leurs sels d'addition avec les bases minérales ou organiques.

4. L'un quelconque des produits de formule (I) dont les noms suivent :
   - la 1-(4-nitrophénylcarbamoyl)-2-cyclopropyl-2-oxopropionitrile,
   - la 1-(4-cyanophénylcarbamoyl)-2-cyclopropyl-2-oxopropionitrile,
   - la 1-(4-chloro 3-méthylphénylcarbamoyl)-2-cyclopropyl-2-oxopropionitrile,
   - la 1-(3-méthyl 4-trifluorométhylphenylcarbamoyl)-2-cyclobutyl-2-oxopropionitrile,
   - le 1-(4-cyano 3-méthylphénylcarbamoyl) 2-cyclopropyl 2-oxopropionitrile,

   ainsi que ses sels d'addition avec les bases minérales ou organiques.

5. Procédé de préparation des nouveaux 3-cycloalkyl-propanamides tels que définis par la formule (I) de la revendication 1, ainsi que de leurs sels, caractérisé en ce que l'on fait réagir un produit de formule (II) :

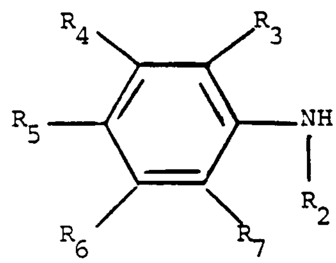

(II)

dans laquelle $R_2$, $R_3$, $R_4$, $R_5$, $R_6$ et $R_7$ ont la signification déja indiquée avec un acide de formule (III) ou un dérivé fonctionnel de cet acide :

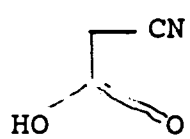

(III)

pour obtenir un produit de formule (IV) :

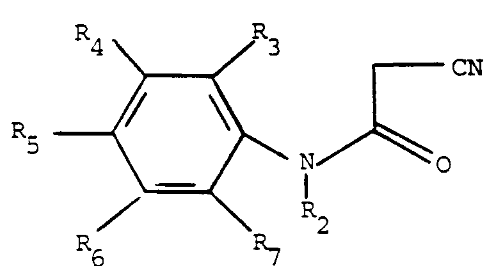

(IV)

dans laquelle $R_2$, $R_3$, $R_4$, $R_5$, $R_6$ et $R_7$ ont la signification déja indiquée, puis fait réagir ce dernier, successivement avec de l'hydrure de sodium, si nécessaire en présence d'un catalyseur tel que l'imidazole puis avec un produit de formule (V):

Hal-CO-$R_1$ (V)

dans laquelle Hal représente un atome d'halogène et $R_1$ a la signification déja indiquée, pour obtenir un produit de formule (I) correspondant que l'on isole et si désiré salifie.

**6.** Procédé de préparation selon la revendication 5, caractérisé en ce que :

- la réaction du produit de formule (II) avec l'acide de formule (III) ou un dérivé fonctionnel de cet acide est effectuée en présence de diisopropylcarbodiimide ou de dicyclohexylcarbodiimide au sein d'un solvant organique anhydre tel que le tétrahydrofuranne ou le dichlorométhane,
- le dérivé fonctionnel de l'acide de formule (III) peut être par exemple le chlorure de cyanoacétyle préparé in situ par action de l'acide cyanoacétique sur le pentachlorure de phosphore,
- la réaction du produit de formule (IV) avec l'hydrure de sodium est effectuée au sein d'un solvant organique anhydre tel que le tétrahydrofuranne.

**7.** Médicaments, caractérisés en ce qu'ils sont constitués par les nouveaux 3-cycloalkyl-propanamides telles que définis par la formule (I) de la revendication 1, ainsi que par leurs sels d'addition avec les acides pharmaceutiquement acceptables.

**8.** Médicaments, caractérisés en ce qu'ils sont constitués par les nouveaux 3-cycloalkyl-propanamides tels que définis à l'une quelconque des revendications 2 à 4, ainsi que par leurs sels d'addition avec les acides pharmaceutiquement acceptables.

**9.** Compositions pharmaceutiques, caractérisées en ce qu'elles renferment à titre de principe actif, l'un au moins des médicaments tels que définis à l'une quelconque des revendications 7 ou 8.

**10.** A titre de produit industriel nouveau, le produit de formule (IV) :

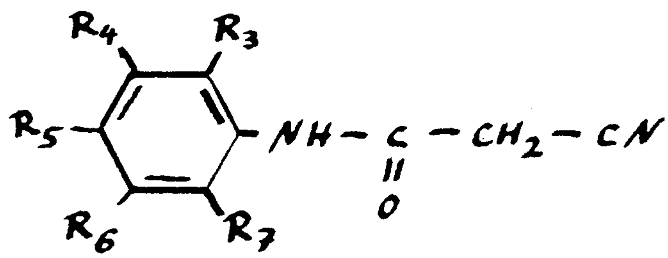

(IV)

dans laquelle
R3, R6, R7 représent un atome d'hydrogène,
R4 représente un radical méthyle et
R5 représente un groupe trifluorométhyl.

**Revendications pour l'Etat contractant suivant : ES**

**1.** Procédé pour préparer des nouveaux 3-cycloalkyl-propanamides répondant à la formule générale (I) :

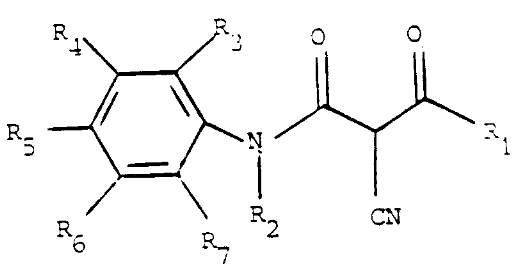

**(I)**

dans laquelle :

- $R_1$ représente un groupement cycloalkyle renfermant de 3 à 6 atomes de carbone,
- $R_2$ représente un atome d'hydrogène, un radical alkyle renfermant de 1 à 3 atomes de carbone,
- $R_3$, $R_4$, $R_5$, $R_6$ et $R_7$, identiques ou différents, représentent un atome d'hydrogène, un atome d'halogène, un radical alkyle linéaire ou ramifié renfermant de 1 à 6 atomes de carbone, un radical alcoxy linéaire ou ramifié renfermant de 1 à 6 atomes de carbone, un radical alkylthio renfermant de 1 à 6 atomes de carbone, un radical $-(CH_2)_m-CF_3$, $-O-(CH_2)_m-CF_3$, $-S-(CH_2)_m-CF_3$

dans lesquels m représente un nombre entier compris entre 0 et 3, un radical $-CF_2$-Hal, $-OCF_2$-Hal,

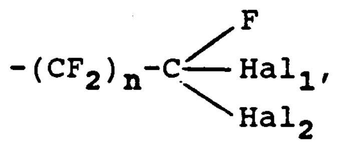

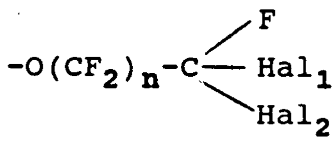, 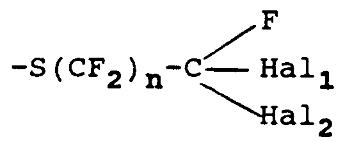

ou $O(CF_2)_n$-CF-$Hal_3$-$CF_3$, dans lequel n représente un nombre entier compris entre 1 et 3, $Hal_1$ et $Hal_2$ identiques ou différents, Hal et $Hal_3$ représentent un atome d'halogène,
ou $R_3$, $R_4$, $R_5$, $R_6$ et $R_7$ identiques ou différents représentent un groupement nitro, un groupement azido, un groupement nitrile, un groupement -CO-R' dans lequel R' représente un radical hydroxy, alkyle ou alcoxy renfermant de 1 à 3 atomes de carbone ou, $R_4$ et $R_5$ forment ensemble un groupement $-O-CH_2-O-$, leurs formes tautomères, ainsi que leurs sels d'addition avec les bases minérales ou organiques, caractérisé en ce que l'on fait réagir un produit de formule (II) :

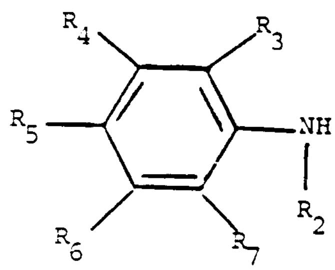

(II)

dans laquelle $R_2$, $R_3$, $R_4$, $R_5$, $R_6$ et $R_7$ ont la signification déja indiquée avec un acide de formule (III) ou un dérivé fonctionnel de cet acide :

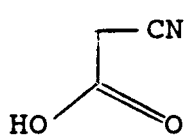

(III)

pour obtenir un produit de formule (IV) :

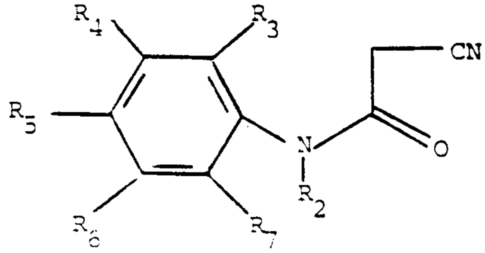

(IV)

dans laquelle $R_2$, $R_3$, $R_4$, $R_5$, $R_6$ et $R_7$ ont la signification déja indiquée, puis fait réagir ce

dernier, successivement avec de l'hydrure de sodium, si nécessaire en présence d'un catalyseur tel que l'imidazole puis avec un produit de formule (V):

Hal-CO-$R_1$ (V)

dans laquelle Hal représente un atome d'halogène et $R_1$ a la signification déja indiquée, pour obtenir un produit de formule (I) correspondant que l'on isole et si désiré salifie.

**2.** Procédé de préparation selon la revendication 1, caractérisé en ce que :
- la réaction du produit de formule (II) avec l'acide de formule (III) ou un dérivé fonctionnel de cet acide est effectuée en présence de diisopropylcarbodiimide ou de dicyclohexylcarbodiimide au sein d'un solvant organique anhydre tel que le tétrahydrofuranne ou le dichlorométhane,
- le dérivé fonctionnel de l'acide de formule (III) peut être par exemple le chlorure de cyanoacétyle préparé in situ par action de l'acide cyanoacétique sur le pentachlorure de phosphore,
- la réaction du produit de formule (IV) avec l'hydrure de sodium est éffectuée au sein d'un solvant organique anhydre tel que le tétrahydrofuranne.

**3.** Procédé selon la revendication 1 ou 2, caractérisé en ce que l'on utilise au départ un produit de formule (II) dans laquelle $R_3$, $R_4$, $R_5$, $R_6$ et $R_7$ identiques ou différents, représentent un atome d'hydrogène, un atome de fluor ,de chlore, de brome ou d'iode, un radical méthyle, éthyle, terbutyle, méthoxy, méthylthio, trifluorométhyle, trifluorométhoxy, trifluorométhylthio, pentafluoroéthyle, bromodifluorométhoxy, acétyle, hydroxycarbonyle, méthoxycarbonyle, nitro, azido, nitrile ou $R_4$ ou $R_5$ forment ensemble un groupement O-$CH_2$-O-, $R_2$ représente un atome d'hydrogène, ou un radical méthyle.

**4.** Procédé selon l'une quelconque des revendications 1 à 3, caractérisé en ce que l'on utilise au départ un produit de formule (II) dans laquelle $R_2$ représente un atome d'hydrogène ou un radical méthyle, $R_3$, $R_4$, $R_5$, $R_6$ et $R_7$ identiques ou différents, représentent un atome d'hydrogène, un atome de fluor, de chlore ou d'iode, un radical méthyle, trifluorométhyle ou nitro et un produit de formule (V) dans laquelle $R_1$ représente un groupement cyclopropyle.

**Revendications pour l'Etat contractant suivant : GR**

**1.** Procédé pour préparer des nouveaux 3-cycloalkyl-propanamides répondant à la formule générale (I) :

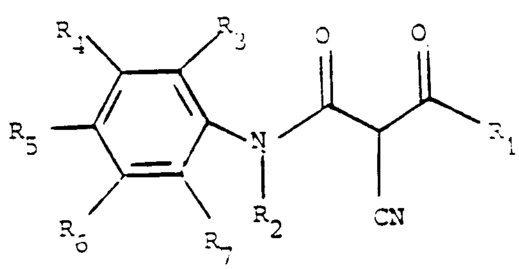

(I)

dans laquelle :
- $R_1$ représente un groupement cycloalkyle renfermant de 3 à 6 atomes de carbone,
- $R_2$ représente un atome d'hydrogène, un radical alkyle renfermant de 1 à 3 atomes de carbone,
- $R_3$, $R_4$, $R_5$, $R_6$ et $R_7$, identiques ou différents, représentent un atome d'hydrogène, un atome d'halogène, un radical alkyle linéaire ou ramifié renfermant de 1 à 6 atomes de carbone, un radical alcoxy linéaire ou ramifié renfermant de 1 à 6 atomes de carbone, un radical alkylthio renfermant de 1 à 6 atomes de carbone, un radical $-(CH_2)_m-CF_3$, $-O-(CH_2)_m-CF_3$, $-S-(CH_2)_m-CF_3$ dans lesquels m représente un nombre entier compris entre 0 et 3, un radical $-CF_2$-Hal, $-OCF_2$-Hal,

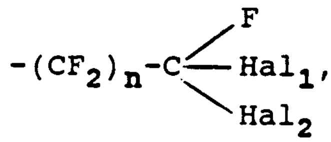

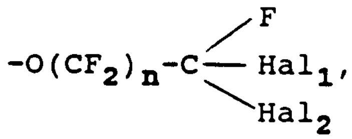 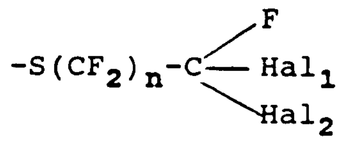

ou $O(CF_2)_n$-CF-$Hal_3$-$CF_3$, dans lequel n représente un nombre entier compris entre 1 et 3, $Hal_1$ et $Hal_2$ identiques ou différents, Hal et $Hal_3$ représentent un atome d'halogène,
ou $R_3$, $R_4$, $R_5$, $R_6$ et $R_7$ identiques ou différents représentent un groupement nitro, un groupement azido, un groupement nitrile, un groupement -CO-R' dans lequel R' représente un radical hydroxy, alkyle ou alcoxy renfermant de 1 à 3 atomes de carbone ou, $R_4$ et $R_5$ forment ensemble un groupement -O-$CH_2$-O-, leurs formes tautomères, ainsi que leurs sels d'addition avec les bases minérales ou organiques, caractérisé en ce que l'on fait réagir un produit de formule (II) :

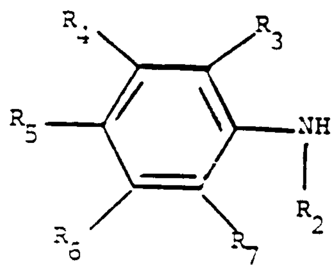

(II)

dans laquelle $R_2$, $R_3$, $R_4$, $R_5$, $R_6$ et $R_7$ ont la signification déja indiquée avec un acide de formule (III) ou un dérivé fonctionnel de cet acide :

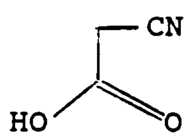

(III)

pour obtenir un produit de formule (IV) :

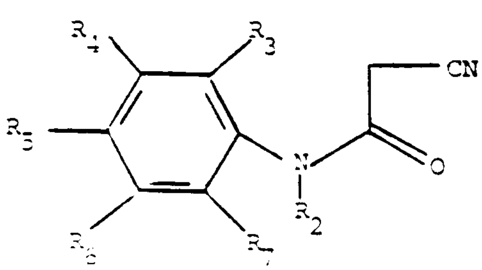

(IV)

dans laquelle $R_2$, $R_3$, $R_4$, $R_5$, $R_6$ et $R_7$ ont la signification déja indiquée, puis fait réagir ce dernier, successivement avec de l'hydrure de sodium, si nécessaire en présence d'un catalyseur tel que l'imidazole puis avec un produit de formule (V):

Hal-CO-$R_1$ (V)

dans laquelle Hal représente un atome d'halogène et $R_1$ a la signification déja indiquée, pour obtenir un produit de formule (I) correspondant que l'on isole et si désiré salifie.

2. Procédé de préparation selon la revendication 1, caractérisé en ce que :
   - la réaction du produit de formule (II) avec l'acide de formule (III) ou un dérivé fonctionnel de cet acide est effectuée en présence de diisopropylcarbodiimide ou de dicyclohexylcarbodiimide au sein d'un solvant organique anhydre tel que le tétrahydrofuranne ou le dichlorométhane,
   - le dérivé fonctionnel de l'acide de formule (III) peut être par exemple le chlorure de cyanoacétyle préparé in situ par action de l'acide cyanoacétique sur le pentachlorure de phosphore,
   - la réaction du produit de formule (IV) avec l'hydrure de sodium est éffectuée au sein d'un solvant organique anhydre tel que le tétrahydrofuranne.

3. Procédé selon la revendication 1 ou 2, caractérisé en ce que l'on utilise au départ un produit de formule (II) dans laquelle $R_3$, $R_4$, $R_5$, $R_6$ et $R_7$ identiques ou différents, représentent un atome d'hydrogène, un atome de fluor ,de chlore, de brome ou d'iode, un radical méthyle, éthyle, terbutyle, méthoxy, méthylthio, trifluorométhyle, trifluorométhoxy, trifluorométhylthio, pentafluoroéthyle, bromodifluorométhoxy, acétyle, hydroxycarbonyle, méthoxycarbonyle, nitro, azido, nitrile ou $R_4$ ou $R_5$ forment ensemble un groupement O-$CH_2$-O-, $R_2$ représente un atome d'hydrogène, ou un radical méthyle.

4. Procédé selon l'une quelconque des revendications 1 à 3, caractérisé en ce que l'on utilise au départ un produit de formule (II) dans laquelle $R_2$ représente un atome d'hydrogène ou un radical méthyle, $R_3$, $R_4$, $R_5$, $R_6$ et $R_7$ identiques ou différents, représentent un atome d'hydrogène, un atome de fluor, de chlore ou d'iode, un radical méthyle, trifluorométhyle ou nitro et un produit de formule (V) dans laquelle $R_1$ représente un groupement cyclopropyle.

5. Procédé selon l'une quelconque des revendications 1 à 4, caractérisé en ce que l'on choisit les produits de formule (II) et (V) de manière telle que l'on prépare l'un quelconque des produits de formule (I) dont les noms suivent :
   - la 1-(4-nitrophénylcarbamoyl)-2-cyclopropyl-2-oxopropionitrile,
   - la 1-(4-cyanophénylcarbamoyl)-2-cyclopropyl-2-oxopropionitrile,
   - la 1-(4-chloro 3-méthylphénylcarbamoyl)-2-cyclopropyl-2-oxopropionitrile,
   - la 1-(3-méthyl 4-trifluorométhylphenylcarbamoyl)-2-cyclobutyl-2-oxopropionitrile,
   - la 1-(4-cyano 3-méthylphénylcarbamoyl) 2-cyclopropyl 2-oxopropionitrile,

   ainsi que ses sels d'addition avec les bases minérales ou organiques.

6. Procédé de préparation de compositions pharmaceutiques, caractérisé en ce que l'on met à titre de principe actif l'un au moins des produits de formule (I) telle que définie à la revendication 1 ou l'un au moins de leurs sels pharmaceutiquement acceptables sous une forme destinée à cet usage.

7. Procédé de préparation de compositions pharmaceutiques, caractérisé en ce que l'on met à titre de principe actif l'un au moins des produits de formule (I) telle que définie à l'une des revendications 2 à 5 ou l'un au moins de leurs sels pharmaceutiquement acceptables sous une forme destinée à cet usage.

8. A titre de produit industriel nouveau, le produit de formule (IV) :

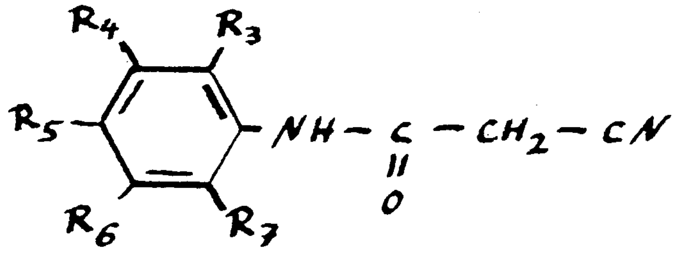

(IV)

dans laquelle

R3, R6, R7 représent un atome d'hydrogène,
R4 représente un radical méthyle et
R5 représente un groupe trifluorométhyl.

## Claims

**Claims for the following Contracting States : AT, BE, CH, DE, DK, FR, GB, IT, LI, LU, NL, SE**

**1.** New 3-cycloalkyl-propanamides corresponding to general formula (I):

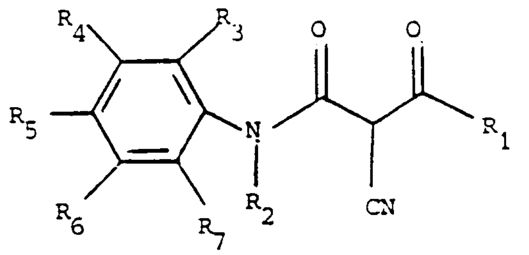

(I)

in which:

- $R_1$ represents a cycloalkyl group containing 3 to 6 carbon atoms,
- $R_2$ represents a hydrogen atom, an alkyl radical containing 1 to 3 carbon atoms,
- $R_3$, $R_4$, $R_5$, $R_6$ and $R_7$, identical or different, represent a hydrogen atom, a halogen atom, a linear or branched alkyl radical containing 1 to 6 carbon atoms, a linear or branched alkoxy radical containing 1 to 6 carbon atoms, an alkylthio radical containing 1 to 6 carbon atoms, a $-(CH_2)_m-CF_3$, $-O-(CH_2)_m-CF_3$, $-S-(CH_2)_m-CF_3$ radical in which m represents an integer comprised between 0 and 3, a $-CF_2-Hal$, $-OCF_2-Hal$,

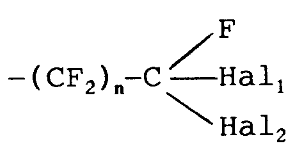 , 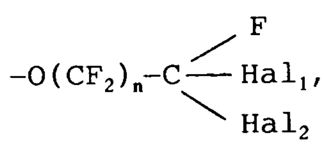 , 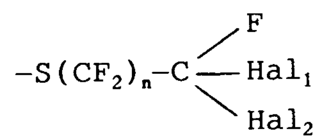

or $O(CF_2)_n-CF-Hal_3-CF_3$ radical in which n represents an integer comprised between 1 and 3, $Hal_1$ and $Hal_2$, identical or different, Hal and $Hal_3$ represent a halogen atom,
or $R_3$, $R_4$, $R_5$, $R_6$ and $R_7$, identical or different, represent a nitro group, an azido group, a nitrile group, a -CO-R' group in which R' represents a hydroxy, alkyl or alkoxy radical containing 1 to 3 carbon atoms or, $R_4$ and $R_5$ form together an $O-CH_2-O-$ group, their tautomeric forms, as well as their addition salts with mineral or organic bases.

**2.** New 3-cycloalkyl-propanamides as defined by formula (I) of claim 1, characterized in that in said formula (I), $R_3$, $R_4$, $R_5$, $R_6$ and $R_7$, identical or different, represent a hydrogen atom, a fluorine, chlorine, bromine or iodine atom, one of the following radicals: methyl, ethyl, terbutyl, methoxy, methylthio, trifluoromethyl, trifluoromethoxy, trifluoromethylthio, pentafluoroethyl, bromodifluoromethoxy, acetyl, hydroxycarbonyl, methoxycarbonyl, nitro, azido, nitrile, or $R_4$ or $R_5$ together form an $O-CH_2-O-$ group, $R_2$ represents a hydrogen atom, or a methyl radical, $R_1$ has the meaning already indicated, as well as their addition salts with mineral or organic bases.

**3.** New 3-cycloalkyl-propanamides corresponding to formula (I) according to claim 1 or 2, characterized in that in said formula (I), $R_1$ represents a cyclopropyl group, $R_2$ represents a hydrogen atom or a methyl radical, $R_3$, $R_4$, $R_5$, $R_6$ and $R_7$, identical or different, represent a hydrogen atom, a fluorine, chlorine or iodine atom, a methyl, trifluoromethyl or nitro radical, as well as their addition salts with mineral or organic bases.

**4.** Any one of the products of formula (I) whose names follow:

- 1-(4-nitroppenylcarbamoyl)-2-cyclopropyl-2-oxopropionitrile,
- 1-(4-cyanophenylcarbamoyl)-2-cyclopropyl-2-oxopropionitrile,

- 1-(4-chloro 3-methylphenylcarbamoyl)-2-cyclopropyl-2-oxopropionitrile,
- 1-(3-methyl 4-trifluoromethylphenylcarbamoyl)-2-cyclobutyl-2-oxopropionitrile,
- 1-(4-cyano 3-methylphenylcarbamoyl) 2-cyclopropyl 2-oxopropionitrile,

as well as its addition salts with mineral or organic acids.

**5.** Preparation process for new 3-cycloalkyl-propanamides as defined by formula (I) of claim 1, as well as for their salts, characterized in that a product of formula (II):

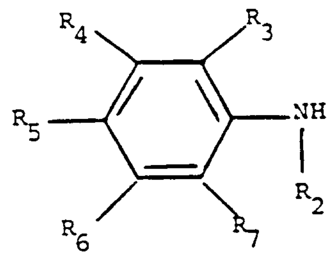

(II)

in which $R_2$, $R_3$, $R_4$, $R_5$, $R_6$ and $R_7$ have the meaning already indicated, is reacted with an acid of formula (III) or a functional derivative of this acid:

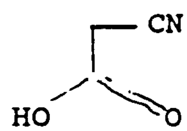

(III)

in order to obtain a product of formula (IV):

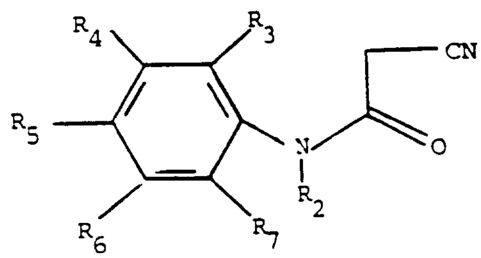

(IV)

in which $R_2$, $R_3$, $R_4$, $R_5$, $R_6$ and $R_7$ have the meaning already indicated, then the latter is reacted, successively with sodium hydride, if necessary in the presence of a catalyst such as imidazole, then with a product of formula (V):

$Hal-CO-R_1$ (V)

in which Hal represents a halogen atom and $R_1$ has the meaning already indicated, in order to obtain a corresponding product of formula (I) which is isolated and if desired salified.

**6.** Preparation process according to claim 5, characterized in that

- the reaction of the product of formula (II) with the acid of formula (III) or a functional derivative of this acid is carried out in the presence of diisopropylcarbodiimide or dicyclohexylcarbodiimide in an anhydrous organic solvent such as tetrahydrofuran or dichloromethane,
- the functional derivative of the acid of formula (III) can be for example cyanoacetyl chloride prepared in situ by the action of cyanoacetic acid on phosphorus pentachloride,
- the reaction of the product of formula (IV) with sodium hydride is carried out in an anhydrous organic solvent such as tetrahydrofuran.

7. Medicaments, characterized in that they are constituted by the new 3-cycloalkyl-propanamides as defined by formula (I) of claim 1, as well as by their addition salts with pharmaceutically acceptable acids.

8. Medicaments, characterized in that they are constituted by the new 3-cycloalkyl-propanamides as defined in any one of claims 2 to 4, as well as by their addition salts with pharmaceutically acceptable acids.

9. Pharmaceutical compositions, characterized in that they contain as active ingredient, at least one of the medicaments as defined in any one of claims 7 or 8.

10. As new industrial products, the product of formula (IV):

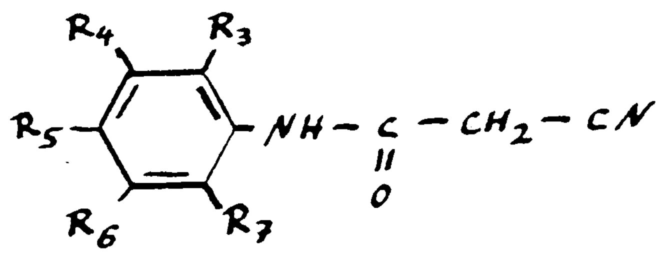

(IV)

in which
$R_3$, $R_6$, $R_7$ represent a hydrogen atom,
$R_4$ represents a methyl radical and
$R_5$ represents a trifluoromethyl group.

**Claims for the following Contracting State : ES**

1. Process for preparing new 3-cycloalkyl-propanamides corresponding to the general formula (I):

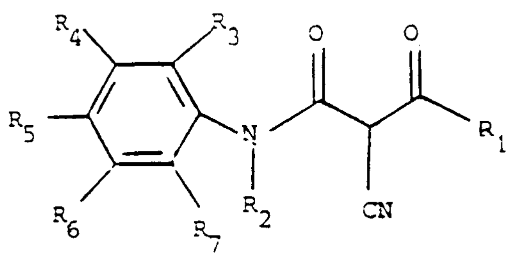

(I)

in which:
- $R_1$ represents a cycloalkyl group containing 3 to 6 carbon atoms,
- $R_2$ represents a hydrogen atom, an alkyl radical containing 1 to 3 carbon atoms,
- $R_3$, $R_4$, $R_5$, $R_6$ and $R_7$, identical or different, represent a hydrogen atom, a halogen atom, a linear or branched alkyl radical containing 1 to 6 carbon atoms, a linear or branched alkoxy radical containing 1 to 6 carbon atoms, an alkylthio radical containing 1 to 6 carbon atoms, a $-(CH_2)_m-CF_3$, $-O-(CH_2)_m-CF_3$, $-S-(CH_2)_m-CF_3$ radical in which m represents an integer comprised between 0 and 3, a $-CF_2$-Hal, $-OCF_2$-Hal,

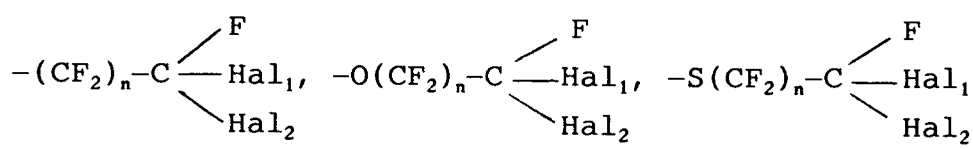

or $O(CF_2)_n$-CF-$Hal_3$-$CF_3$ radical in which n represents an integer comprised between 1 and 3,

$Hal_1$ and $Hal_2$, identical or different, Hal and $Hal_3$ represent a halogen atom, or $R_3$, $R_4$, $R_5$, $R_6$ and $R_7$, identical or different, represent a nitro group, an azido group, a nitrile group, a -CO-R' group in which R' represents a hydroxy, alkyl or alkoxy radical containing 1 to 3 carbon atoms or, $R_4$ and $R_5$ form together an $O-CH_2-O-$ group, their tautomeric forms, as well as their addition salts with mineral or organic bases, characterized in that a product of formula (II):

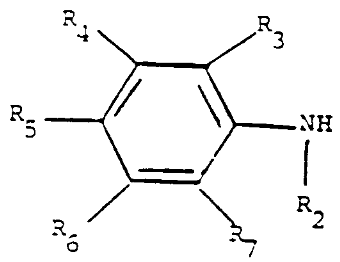

(II)

in which $R_2$, $R_3$, $R_4$ $R_5$, $R_6$ and $R_7$ have the meaning already indicated, is reacted with an acid of formula (III) or a functional derivative of this acid:

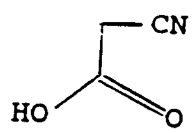

(III)

in order to obtain a product of formula (IV):

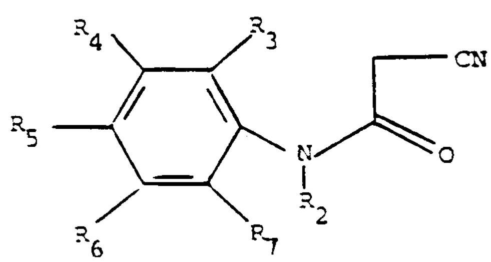

(IV)

in which $R_2$, $R_3$, $R_4$, $R_5$, $R_6$ and $R_7$ have the meaning already indicated, then the latter is reacted, successively with sodium hydride, if necessary in the presence of a catalyst such as imidazole, then with a product of formula (V):

$Hal-CO-R_1$ (V)

in which Hal represents a halogen atom and $R_1$ has the meaning already indicated, in order to obtain a corresponding product of formula (I) which is isolated and if desired salified.

**2.** Preparation process according to claim 1, characterized in that:
- the reaction of the product of formula (II) with the acid of formula (III) or a functional derivative of this acid is carried out in the presence of diisopropylcarbodiimide or dicyclohexylcarbodiimide in an anhydrous organic solvent such as tetrahydrofuran or dichloromethane,
- the functional derivative of the acid of formula (III) can be for example cyanoacetyl chloride prepared in situ by the action of cyanoacetic acid on phosphorus pentachloride,
- the reaction of the product of formula (IV) with sodium hydride is carried out in an anhydrous organic solvent such as tetrahydrofuran.

**3.** Process according to claim 1 or 2, characterized in that a product of formula (II) is used at the start in which $R_3$, $R_4$, $R_5$, $R_6$ and $R_7$, identical or different, represent a hydrogen atom, a fluorine, chlorine,

bromine or iodine atom, one of the following radicals: methyl, ethyl, terbutyl, methoxy, methylthio, trifluoromethyl, trifluoromethyl, trifluoromethoxy, trifluoromethylthio, pentafluoroethyl, bromodifluoromethoxy, acetyl, hydroxycarbonyl, methoxycarbonyl, nitro, azido, nitrile, or $R_4$ or $R_5$ together form an $O-CH_2-O-$ group, $R_2$ represents a hydrogen atom, or a methyl radical.

**4.** Process according to any one of claims 1 to 3, characterized in that a product of formula (II) is used at the start in which $R_2$ represents a hydrogen atom or a methyl radical, $R_3$, $R_4$, $R_5$, $R_6$ and $R_7$, identical or different, represent a hydrogen atom, a fluorine, chlorine or iodine atom, a methyl, trifluoromethyl or nitro radical and a product of formula (V) is used in which $R_1$ represents a cyclopropyl group.

**Claims for the following Contracting State : GR**

**1.** Process for preparing new 3-cycloalkyl-propanamides corresponding to the general formula (I):

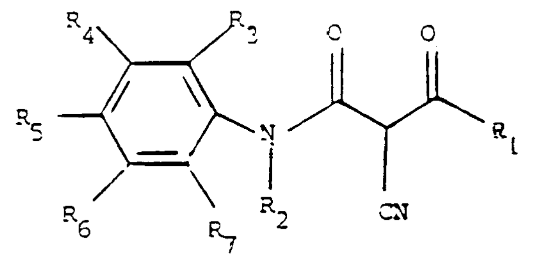

(I)

in which:

- $R_1$ represents a cycloalkyl group containing 3 to 6 carbon atoms,
- $R_2$ represents a hydrogen atom, an alkyl radical containing 1 to 3 carbon atoms,
- $R_3$, $R_4$, $R_5$, $R_6$ and $R_7$, identical or different, represent a hydrogen atom, a halogen atom, a linear or branched alkyl radical containing 1 to 6 carbon atoms, a linear or branched alkoxy radical containing 1 to 6 carbon atoms, an alkylthio radical containing 1 to 6 carbon atoms, a $-(CH_2)_m-CF_3$, $-O-(CH_2)_m-CF_3$, $-S-(CH_2)_m-CF_3$ radical in which m represents an integer comprised between 0 and 3, a $-CF_2-Hal$, $-OCF_2-Hal$,

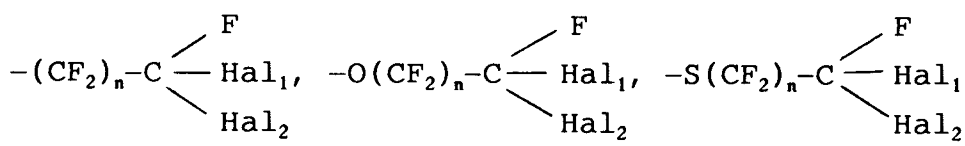

or $O(CF_2)_n-CF-Hal_3-CF_3$ radical in which n represents an integer comprised between 1 and 3, $Hal_1$ and $Hal_2$, identical or different, Hal and $Hal_3$ represent a halogen atom,

or $R_3$, $R_4$, $R_5$, $R_6$ and $R_7$, identical or different, represent a nitro group, an azido group, a nitrile group, a -CO-R' group in which R' represents a hydroxy, alkyl or alkoxy radical containing 1 to 3 carbon atoms or, $R_4$ and $R_5$ form together an $O-CH_2-O-$ group, their tautomeric forms, as well as their addition salts with mineral or organic bases, characterized in that a product of formula (II):

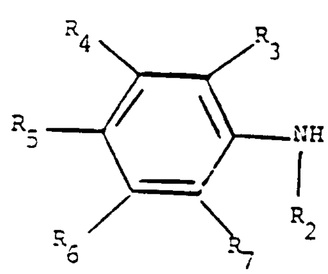

(II)

in which $R_2$, $R_3$, $R_4$, $R_5$, $R_6$ and $R_7$ have the meaning already indicated, is reacted with an acid of formula (III) or a functional derivative of this acid:

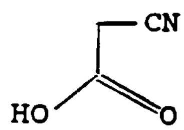

(III)

in order to obtain a product of formula (IV):

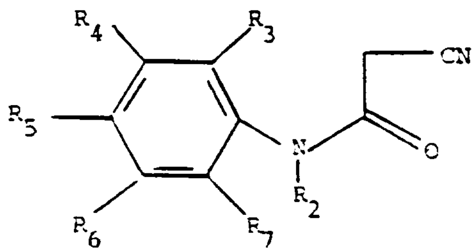

(IV)

in which $R_2$, $R_3$, $R_4$, $R_5$, $R_6$ and $R_7$ have the meaning already indicated, then the latter is reacted, successively with sodium hydride, if necessary in the presence of a catalyst such as imidazole, then with a product of formula (V):

Hal-CO-$R_1$ (V)

in which Hal represents a halogen atom and $R_1$ has the meaning already indicated, in order to obtain a corresponding product of formula (I) which is isolated and if desired salified.

**2.** Preparation process according to claim 1, characterized in that:
- the reaction of the product of formula (II) with the acid of formula (III) or a functional derivative of this acid is carried out in the presence of diisopropylcarbodiimide or dicyclohexylcarbodiimide in an anhydrous organic solvent such as tetrahydrofuran or dichloromethane,
- the functional derivative of the acid of formula (III) can be for example cyanoacetyl chloride prepared in situ by the action of cyanoacetic acid on phosphorus pentachloride,
- the reaction of the product of formula (IV) with sodium hydride is carried out in an anhydrous organic solvent such as tetrahydrofuran.

**3.** Process according to claim 1 or 2, characterized in that a product of formula (II) is used at the start in which $R_3$, $R_4$, $R_5$, $R_6$ and $R_7$, identical or different, represent a hydrogen atom, a fluorine, chlorine, bromine or iodine atom, one of the following radicals: methyl, ethyl, terbutyl, methoxy, methylthio, trifluoromethyl, trifluoromethyl, trifluoromethoxy, trifluoromethylthio, pentafluoroethyl, bromodifluoromethoxy, acetyl, hydroxycarbonyl, methoxycarbonyl, nitro, azido, nitrile, or $R_4$ or $R_5$ together form an O-$CH_2$-O- group, $R_2$ represents a hydrogen atom, or a methyl radical.

**4.** Process according to any one of claims 1 to 3, characterized in that a product of formula (II) is used at the start in which $R_2$ represents a hydrogen atom or a methyl radical, $R_3$, $R_4$, $R_5$, $R_6$ and $R_7$, identical or different, represent a hydrogen atom, a fluorine, chlorine or iodine atom, a methyl, trifluoromethyl or nitro radical and a product of formula (V) is used in which $R_1$ represents a cyclopropyl group.

**5.** Process according to any one of claims 1 to 4, characterized in that the products of formula (II) and (V) are chosen in such a way that any one of the products of formula (I) of which the names follow are prepared:
- 1-(4-nitrophenylcarbamoyl)-2-cyclopropyl-2-oxopropionitrile,
- 1-(4-cyanophenylcarbamoyl)-2-cyclopropyl-2-oxopropionitrile,
- 1-(4-chloro 3-methylphenylcarbamoyl)-2-cyclopropyl-2-oxopropionitrile,
- 1-(3-methyl 4-trifluoromethylphenylcarbamoyl)-2-cyclobutyl-2-oxopropionitrile,
- 1-(4-cyano 3-methylphenylcarbamoyl) 2-cyclopropyl 2-oxopropionitrile,

as well as its addition salts with mineral or organic bases.

6. Preparation process for pharmaceutical compositions, characterized in that at least one of the products of formula (I) as defined in claim 1 or at least one of their pharmaceutically acceptable salts is used as active ingredient in a form intended for this use.

7. Preparation process for pharmaceutical compositions, characterized in that at least one of the products of formula (I) as defined in one of claims 2 to 5 or at least one of their pharmaceutically acceptable salts is used as active ingredient in a form intended for this use.

8. As a new industrial product, the product of formula (IV):

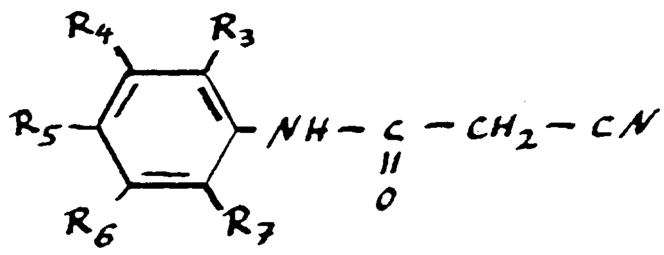

(IV)

in which
$R_3$, $R_6$, $R_7$ represent a hydrogen atom,
$R_4$ represents a methyl radical and
$R_5$ represents a trifluoromethyl group.

**Patentansprüche**
**Patentansprüche für folgende Vertragsstaaten : AT, BE, CH, DE, DK, FR, GB, IT, LI, LU, NL, SE**

1. Neue 3-Cycloalkylpropanamide entsprechend der allgemeinen Formel (I):

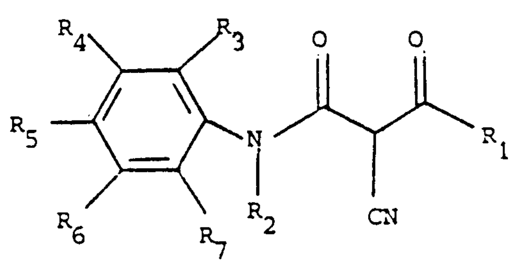

(I)

worin:
$R_1$ eine Cycloalkylgruppe mit 3 bis 6 Kohlenstoffatomen bedeutet,
$R_2$ bedeutet ein Wasserstoffatom, einen Alkylrest mit 1 bis 3 Kohlenstoffatomen,
$R_3$, $R_4$, $R_5$, $R_6$ und $R_7$, die identisch oder verschieden sind, bedeuten ein Wasserstoffatom, ein Halogenatom, einen geraden oder verzweigten Alkylrest mit 1 bis 6 Kohlenstoffatomen, einen geraden oder verzweigten Alkoxyrest mit 1 bis 6 Kohlenstoffatomen, einen Alkylthiorest mit 1 bis 6 Kohlenstoffatomen, einen Rest $-(CH_2)_m-CF_3$, $-O-(CH_2)_m-CF_3$, $-S-(CH_2)_m-CF_3$, worin m eine ganze Zahl zwischen 0 und 3 bedeutet, einen Rest $-CF_2-Hal$, $-OCF_2-Hal$,

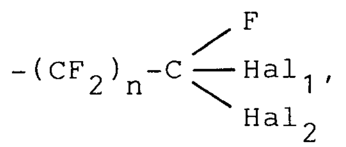

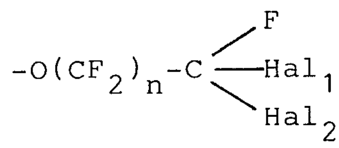

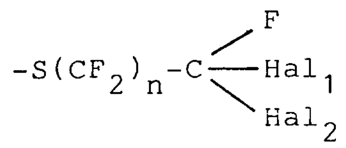

oder $O(CF_2)_n-CF-Hal_3-CF_3$ worin n eine ganze Zahl zwischen 1 und 3 bedeutet, $Hal_1$ und $Hal_2$, die identisch oder verschieden sind, Hal und $Hal_3$ bedeuten ein Halogenatom, oder $R_3$, $R_4$, $R_5$, $R_6$ und $R_7$, die identisch oder verschieden sind, bedeuten eine Nitrogruppe, eine Azidogruppe, eine Nitrilgruppe, eine Gruppe -CO-R', worin R' einen Hydroxy-, Alkyl- oder Alkoxyrest mit 1 bis 3 Kohlenstoffatomen darstellt, oder $R_4$ und $R_5$ bilden zusammen eine Gruppe $-O-CH_2-O-$, ihre tautomeren Formen sowie ihre Additionssalze mit mineralischen oder organischen Basen.

**2.** Neue 3-Cycloalkylpropanamide wie in Formel (I) von Anspruch 1 definiert, dadurch gekennzeichnet, daß in dieser Formel (I) $R_3$, $R_4$, $R_5$, $R_6$ und $R_7$, die identisch oder verschieden sind, ein Wasserstoffatom, ein Fluor-, Chlor-, Brom- oder Jodatom bedeuten, einen Methyl-, Ethyl-, tert.-Butyl-, Methoxy-, Methylthio-, Trifluormethyl-, Trifluormethoxy-, Trifluormethylthio-, Pentafluorethyl-, Bromdifluormethoxy-, Acetyl-, Hydroxycarbonyl-, Methoxycarbonyl-, Nitro-, Azido-, Nitrilrest bedeuten, oder $R_4$ oder $R_5$ bilden zusammen eine Gruppe $-O-CH_2-O-$, $R_2$ bedeutet ein Wasserstoffatom oder einen Methylrest, $R_1$ hat die bereits angegebene Bedeutung sowie ihre Additionssalze mit mineralischen oder organischen Basen.

**3.** Neue 3-Cycloalkylpropanamide entsprechend der Formel (I) gemäß Anspruch 1 oder 2, dadurch gekennzeichnet, daß in dieser Formel (I) $R_1$ eine Cyclopropylgruppe bedeutet, $R_2$ ein Wasserstoffatom oder einen Methylrest darstellt, $R_3$, $R_4$, $R_5$, $R_6$ und $R_7$, die identisch oder verschieden sind, ein Wasserstoff-, Fluor-, Chlor- oder Jodatom, einen Methyl-, Trifluormethyl- oder Nitrorest bedeuten, sowie ihre Additionssalze mit mineralischen oder organischen Basen.

**4.** Eines der Produkte der Formel (I) mit den folgenden Bezeichnungen:
1-(4-Nitrophenylcarbamoyl)-2-cyclopropyl-2-oxopropionitril,
1-(4-Cyanophenylcarbamoyl)-2-cyclopropyl-2-oxopropionitril,
1-(4-Chlor-3-methylphenylcarbamoyl)-2-cyclopropyl-2-oxopropionitril,
1-(3-Methyl-4-trifluormethylphenylcarbamoyl)-2-cyclobutyl-2-oxopropionitril,
1-(4-Cyano-3-methylphenylcarbamoyl)-2-cyclopropyl-2-oxopropionitril,
sowie ihre Additionssalze mit mineralischen oder organischen Basen.

**5.** Verfahren zur Herstellung der neuen 3-Cycloalkylpropanamide, wie sie durch die Formel (I) von Anspruch 1 definiert sind, sowie ihrer Salze, dadurch gekennzeichnet daß man ein Produkt der Formel (II)

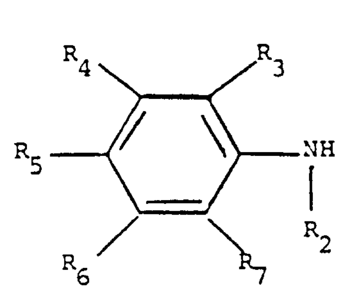

(II)

worin $R_2$, $R_3$, $R_4$, $R_5$, $R_6$ und $R_7$, die bereits angegebene Bedeutung haben, mit einer Säure der

Formel (III) oder einem funktionellen Derivat dieser Säure

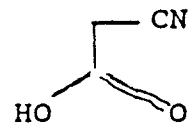

(III)

zur Reaktion bringt, um ein Produkt der Formel (IV)

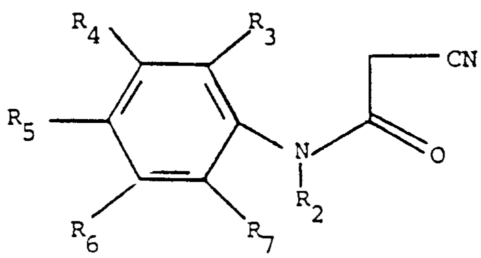

(IV)

zu erhalten, worin $R_2$, $R_3$, $R_4$, $R_5$, $R_6$ und $R_7$ die bereits angegebene Bedeutung haben, dann dieses letztere nacheinander mit Natriumhydrid, falls notwendig in Gegenwart eines Katalysators wie Imidazol zur Reaktion bringt und dann mit einem Produkt der Formel (V):

Hal-CO-$R_1$ (V)

worin Hal ein Halogenatom bedeutet und $R_1$ die bereits angegebene Bedeutung hat, um ein entsprechendes Produkt der Formel (I) zu erhalten, das man isoliert und gegebenenfalls in das Salz überführt.

**6.** Herstellungsverfahren gemäß Anspruch 5, dadurch gekennzeichnet, daß
- die Reaktion des Produkts der Formel (II) mit der Säure der Formel (III) oder einem funktionellen Derivat dieser Säure in Gegenwart von Diisopropylcarbodiimid oder Dicyclohexylcarbodiimid in einem wasserfreien organischen Lösungsmittel wie Tetrahydrofuran oder Dichlormethan bewirkt wird;
- das funktionelle Derivat der Säure der Formel (III) beispielsweise das Cyanoacetylchlorid sein kann, das in situ durch Einwirkung von Cyanoessigsäure auf Phosphorpentachlorid hergestellt wird;
- die Reaktion des Produkts der Formel (IV) mit dem Natriumhydrid in einem wasserfreien organischen Lösungsmittel wie Tetrahydrofuran bewirkt wird.

**7.** Arzneimittel, dadurch gekennzeichnet, daß sie aus den neuen 3-Cycloalkylpropanamiden bestehen, wie sie durch Formel (I) von Anspruch 1 definiert sind, sowie ihre Additionssalze mit pharmazeutisch annehmbaren Säuren.

**8.** Arzneimittel, dadurch gekennzeichnet, daß sie aus den neuen 3-Cycloalkylpropanamiden bestehen, wie sie in einem der Ansprüche 2 bis 4 definiert sind, sowie aus ihren Salzen mit pharmazeutisch annehmbaren Säuren.

**9.** Pharmazeutische Zusammensetzungen, dadurch gekennzeichnet, daß sie als Wirkstoff mindestens eines der Arzneimittel enthalten, wie sie in einem der Ansprüche 7 oder 8 definiert sind.

**10.** Das Produkt der Formel (IV) als neues industrielles Produkt

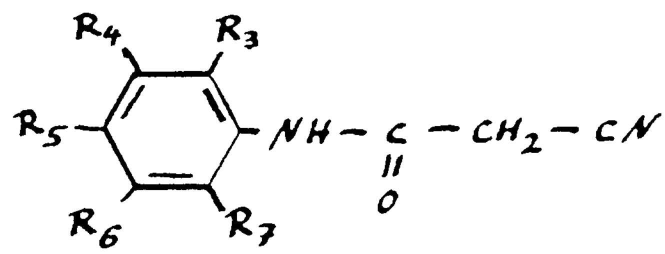

(IV)

worin
$R_3$, $R_6$, $R_7$ ein Wasserstoffatom bedeuten,
$R_4$ einen Methylrest darstellt, und
$R_5$ eine Trifluoromethylgruppe bedeutet.

**Patentansprüche für folgenden Vertragsstaat : ES**

**1.** Verfahren zur Herstellung von neuen 3-Cycloalkylpropanamiden entsprechend der allgemeinen Formel (I):

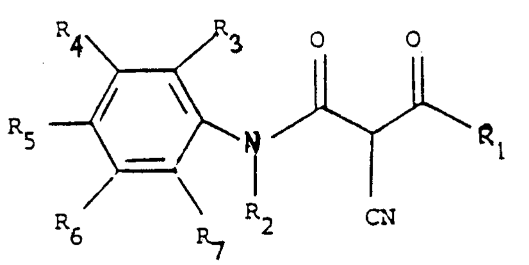

(I)

worin:
$R_1$ eine Cycloalkylgruppe mit 3 bis 6 Kohlenstoffatomen bedeutet,
$R_2$ bedeutet ein Wasserstoffatom, einen Alkylrest mit 1 bis 3 Kohlenstoffatomen,
$R_3$, $R_4$, $R_5$, $R_6$ und $R_7$, die identisch oder verschieden sind, bedeuten ein Wasserstoffatom, ein Halogenatom, einen geraden oder verzweigten Alkylrest mit 1 bis 6 Kohlenstoffatomen, einen geraden oder verzweigten Alkoxyrest mit 1 bis 6 Kohlenstoffatomen, einen Alkylthiorest mit 1 bis 6 Kohlenstoffatomen, einen Rest $-(CH_2)_m-CF_3$, $-O-(CH_2)_m-CF_3$, $-S-(CH_2)_m-CF_3$, worin m eine ganze Zahl zwischen 0 und 3 bedeutet, einen Rest $-CF_2-Hal$, $-OCF_2-Hal$,

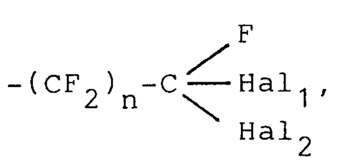,

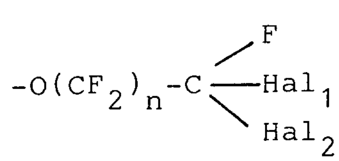

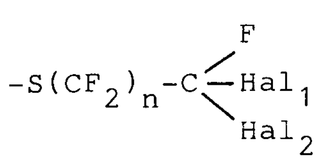

oder $O(CF_2)_n-CF-Hal_3-CF_3$ worin n eine ganze Zahl zwischen 1 und 3 bedeutet, $Hal_1$ und $Hal_2$, die identisch oder verschieden sind, Hal und $Hal_3$ bedeuten ein Halogenatom,

oder $R_3$, $R_4$, $R_5$, $R_6$ und $R_7$, die identisch oder verschieden sind, bedeuten eine Nitrogruppe, eine Azidogruppe, eine Nitrilgruppe, eine Gruppe -CO-R', worin R' einen Hydroxy-, Alkyl- oder Alkoxyrest mit 1 bis 3 Kohlenstoffatomen darstellt, oder $R_4$ und $R_5$ bilden zusammen eine Gruppe $-O-CH_2-O-$, ihre tautomeren Formen sowie ihre Additionssalze mit mineralischen oder organischen Basen, dadurch gekennzeichnet, daß man ein Produkt der Formel (II)

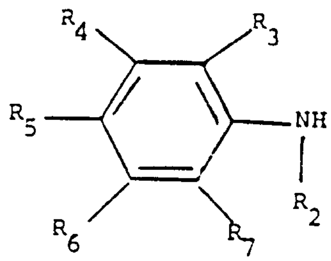

(II)

worin $R_2$, $R_3$, $R_4$, $R_5$, $R_6$ und $R_7$, die bereits angegebene Bedeutung haben, mit einer Säure der Formel (III) oder einem funktionellen Derivat dieser Säure

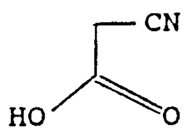

(III)

zur Reaktion bringt, um ein Produkt der Formel (IV)

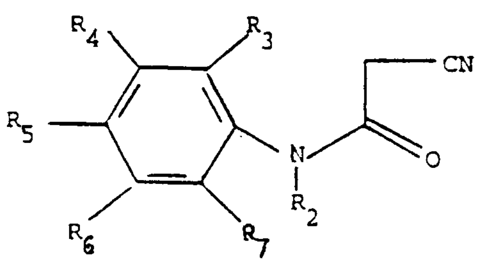

(IV)

zu erhalten, worin $R_2$, $R_3$, $R_4$, $R_5$, $R_6$ und $R_7$ die bereits angegebene Bedeutung haben, dann dieses letztere nacheinander mit Natriumhydrid, falls notwendig in Gegenwart eines Katalysators wie Imidazol zur Reaktion bringt und dann mit einem Produkt der Formel (V):

$Hal-CO-R_1$ (V)

worin Hal ein Halogenatom bedeutet und $R_1$ die bereits angegebene Bedeutung hat, um ein entsprechendes Produkt der Formel (I) zu erhalten, das man isoliert und gegebenenfalls in das Salz überführt.

**2.** Herstellungsverfahren gemäß Anspruch 1, dadurch gekennzeichnet, daß
- die Reaktion des Produkts der Formel (II) mit der Säure der Formel (III) oder einem funktionellen Derivat dieser Säure in Gegenwart von Diisopropylcarbodiimid oder Dicyclohexylcarbodiimid in einem wasserfreien organischen Lösungsmittel wie Tetrahydrofuran oder Dichlormethan bewirkt wird;
- das funktionelle Derivat der Säure der Formel (III) beispielsweise das Cyanoacetylchlorid sein kann, das in situ durch Einwirkung von Cyanoessigsäure auf Phosphorpentachlorid hergestellt wird;
- die Reaktion des Produkts der Formel (IV) mit dem Natriumhydrid in einem wasserfreien organischen Lösungsmittel wie Tetrahydrofuran bewirkt wird.

**3.** Verfahren gemäß Anspruch 1 oder 2, dadurch gekennzeichnet, daß man zu Beginn ein Produkt der Formel (II) verwendet, worin $R_3$, $R_4$, $R_5$, $R_6$ und $R_7$, die identisch oder verschieden sind, ein Wasserstoffatom, ein Fluor-, Chlor-, Brom- oder Jodatom bedeutet, einen Methyl-, Ethyl-, tert.-Butyl-, Methoxy-, Methylthio-, Trifluormethyl-, Trifluormethoxy-, Trifluormethylthio-, Pentafluorethyl-, Bromdifluormethoxy-, Acetyl-, Hydroxycarbonyl-, Methoxycarbonyl-, Nitro-, Azido-, Nitrilrest bedeuten, oder $R_4$ oder $R_5$ bilden zusammen eine Gruppe $-O-CH_2-O-$, $R_2$ bedeutet ein Wasserstoffatom oder einen Methylrest.

**4.** Verfahren gemäß einem der Ansprüche 1 bis 3, dadurch gekennzeichnet, daß man zu Beginn ein Produkt der Formel (II) verwendet, worin $R_2$ ein Wasserstoffatom oder einen Methylrest darstellt, $R_3$, $R_4$, $R_5$, $R_6$ und $R_7$, die identisch oder verschieden sind, bedeuten ein Wasserstoff-, Fluor-, Chlor- oder Jodatom, einen Methyl-, Trifluormethyl- oder Nitrorest, und ein Produkt der Formel (V), worin $R_1$ eine Cyclopropylgruppe darstellt.

**Patentansprüche für folgenden Vertragsstaat : GR**

**1.** Verfahren zur Herstellung von neuen 3-Cycloalkylpropanamiden entsprechend der allgemeinen Formel (I):

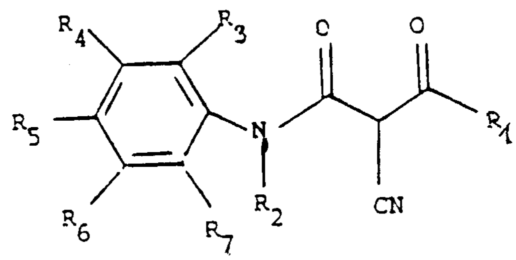

(I)

worin:
$R_1$ eine Cycloalkylgruppe mit 3 bis 6 Kohlenstoffatomen bedeutet,
$R_2$ bedeutet ein Wasserstoffatom, einen Alkylrest mit 1 bis 3 Kohlenstoffatomen,
$R_3$, $R_4$, $R_5$, $R_6$ und $R_7$, die identisch oder verschieden sind, bedeuten ein Wasserstoffatom, ein Halogenatom, einen geraden oder verzweigten Alkylrest mit 1 bis 6 Kohlenstoffatomen, einen geraden oder verzweigten Alkoxyrest mit 1 bis 6 Kohlenstoffatomen, einen Alkylthiorest mit 1 bis 6 Kohlenstoffatomen, einen Rest $-(CH_2)_m-CF_3$, $-O-(CH_2)_m-CF_3$, $-S-(CH_2)_m-CF_3$, worin m eine ganze Zahl zwischen 0 und 3 bedeutet, einen Rest $-CF_2-Hal$, $-OCF_2-Hal$,

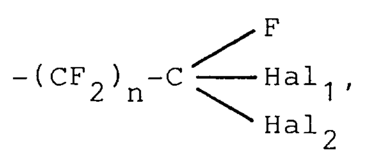

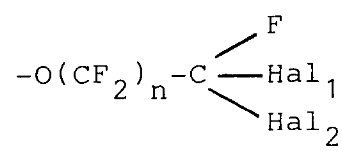

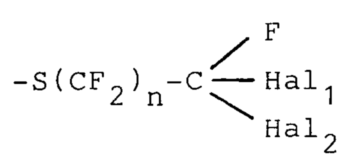

oder $O(CF_2)_n-CF-Hal_3-CF_3$ worin n eine ganze Zahl zwischen 1 und 3 bedeutet, $Hal_1$ und $Hal_2$, die identisch oder verschieden sind, Hal und $Hal_3$ bedeuten ein Halogenatom,
oder $R_3$, $R_4$, $R_5$, $R_6$ und $R_7$, die identisch oder verschieden sind, bedeuten eine Nitrogruppe, eine Azidogruppe, eine Nitrilgruppe, eine Gruppe -CO-R', worin R' einen Hydroxy-, Alkyl- oder Alkoxyrest mit 1 bis 3 Kohlenstoffatomen darstellt, oder $R_4$ und $R_5$ bilden zusammen eine Gruppe $-O-CH_2-O-$, ihre tautomeren Formen sowie ihre Additionssalze mit mineralischen oder organischen Basen, dadurch

gekennzeichnet, daß man ein Produkt der Formel (II)

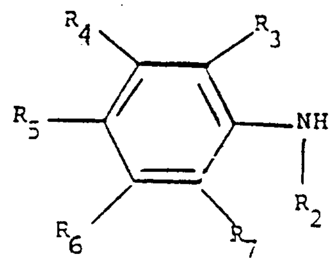

(II)

worin $R_2$, $R_3$, $R_4$, $R_5$, $R_6$ und $R_7$, die bereits angegebene Bedeutung haben, mit einer Säure der Formel (III) oder einem funktionellen Derivat dieser Säure

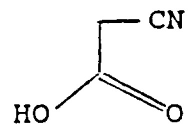

(III)

zur Reaktion bringt, um ein Produkt der Formel (IV)

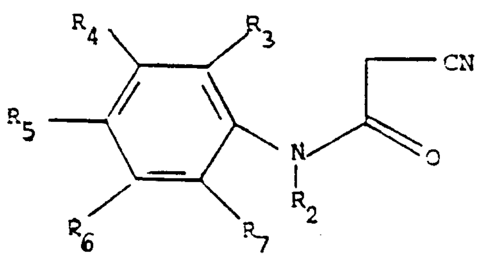

(IV)

zu erhalten, worin $R_2$, $R_3$, $R_4$, $R_5$, $R_6$ und $R_7$ die bereits angegebene Bedeutung haben, dann dieses letztere nacheinander mit Natriumhydrid, falls notwendig in Gegenwart eines Katalysators wie Imidazol zur Reaktion bringt und dann mit einem Produkt der Formel (V):

Hal-CO-$R_1$ (V)

umsetzt, worin Hal ein Halogenatom bedeutet und $R_1$ die bereits angegebene Bedeutung hat, um ein entsprechendes Produkt der Formel (I) zu erhalten, das man isoliert und gegebenenfalls in das Salz überführt.

**2.** Herstellungsverfahren gemäß Anspruch 1, dadurch gekennzeichnet, daß
- die Reaktion des Produkts der Formel (II) mit der Säure der Formel (III) oder einem funktionellen Derivat dieser Säure in Gegenwart von Diisopropylcarbodiimid oder Dicyclohexylcarbodiimid in einem wasserfreien organischen Lösungsmittel wie Tetrahydrofuran oder Dichlormethan bewirkt wird;
- das funktionelle Derivat der Säure der Formel (III) beispielsweise das Cyanoacetylchlorid sein kann, das in situ durch Einwirkung von Cyanoessigsäure auf Phosphorpentachlorid hergestellt wird;
- die Reaktion des Produkts der Formel (IV) mit dem Natriumhydrid in einem wasserfreien organischen Lösungsmittel wie Tetrahydrofuran bewirkt wird.

**3.** Verfahren gemäß Anspruch 1 oder 2, dadurch gekennzeichnet, daß man zu Beginn ein Produkt der Formel (II) verwendet, worin $R_3$, $R_4$, $R_5$, $R_6$ und $R_7$, die identisch oder verschieden sind, ein

Wasserstoffatom, ein Fluor-, Chlor-, Brom- oder Jodatom bedeuten, einen Methyl-, Ethyl-, tert.-Butyl-, Methoxy-, Methylthio-, Trifluormethyl-, Trifluormethoxy-, Trifluormethylthio-, Pentafluorethyl-, Bromdifluormethoxy-, Acetyl-, Hydroxycarbonyl-, Methoxycarbonyl-, Nitro-, Azido-, Nitrilrest bedeuten, oder $R_4$ oder $R_5$ bilden zusammen eine Gruppe $-O-CH_2-O-$, $R_2$ bedeutet ein Wasserstoffatom oder einen Methylrest.

4. Verfahren gemäß einem der Ansprüche 1 bis 3, dadurch gekennzeichnet, daß man zu Beginn ein Produkt der Formel (II) verwendet, worin $R_2$ ein Wasserstoffatom oder einen Methylrest darstellt, $R_3$, $R_4$, $R_5$, $R_6$ und $R_7$, die identisch oder verschieden sind, ein Wasserstoff-, Fluor-, Chlor- oder Jodatom, einen Methyl-, Trifluormethyl- oder Nitrorest bedeuten, und ein Produkt der Formel (V) verwendet, worin $R_1$ eine Cyclopropylgruppe darstellt.

5. Verfahren gemäß einem der Ansprüche 1 bis 4, dadurch gekennzeichnet, daß man die Produkte der Formel (II) und (V) derart auswählt, daß man eines der Produkte der Formel (I) mit den folgenden Bezeichnungen herstellt:
1-(4-Nitrophenylcarbamoyl)-2-cyclopropyl-2-oxopropionitril,
1-(4-Cyanophenylcarbamoyl)-2-cyclopropyl-2-oxopropionitril,
1-(4-Chlor-3-methylphenylcarbamoyl)-2-cyclopropyl-2-oxopropionitril,
1-(3-Methyl-4-trifluormethylphenylcarbamoyl)-2-cyclobutyl-2-oxopropionitril,
1-(4-Cyano-3-methylphenylcarbamoyl)-2-cyclopropyl-2-oxopropionitril,
sowie ihre Additionssalze mit mineralischen oder organischen Basen.

6. Verfahren zur Herstellung von pharmazeutischen Zusammensetzungen, dadurch gekennzeichnet, daß man als Wirkstoff mindestens eines der Produkte der Formel (I), wie in Anspruch 1 definiert, oder mindestens eines seiner pharmazeutisch annehmbaren Salze in einer Form einsetzt, die für diesen Verwendungszweck geeignet ist.

7. Verfahren zur Herstellung von pharmazeutischen Zusammensetzungen, dadurch gekennzeichnet, daß man als Wirkstoff mindestens eines der Produkte der Formel (I), wie in einem der Ansprüche 2 bis 5 definiert, oder mindestens eines seiner pharmazeutisch annehmbaren Salze in einer Form einsetzt, die für diesen Verwendungszweck geeignet ist.

8. Das Produkt der Formel (IV) als neues industrielles Produkt

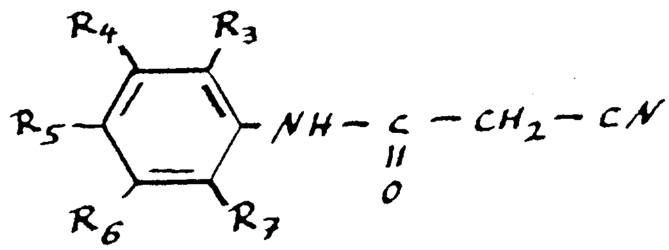

(IV)

worin
$R_3$, $R_6$, $R_7$ ein Wasserstoffatom bededuten,
$R_4$ einen Methylrestrest darstellt, und
$R_5$ eine Trifluoromethylgruppe bedeutet.